(19) Europäisches Patentamt
European Patent Office
Office européen des brevets

(11) Publication number: **0 489 933 A1**

(12) **EUROPEAN PATENT APPLICATION published in accordance with Art. 158(3) EPC**

(21) Application number: **91912082.4**

(51) Int. Cl.[5]: **C07J 9/00**, A61K 31/575

(22) Date of filing: **01.07.91**

(86) International application number: **PCT/JP91/00888**

(87) International publication number: **WO 92/00316 (09.01.92 92/02)**

(30) Priority: **30.06.90 JP 171354/90**

(43) Date of publication of application: **17.06.92 Bulletin 92/25**

(84) Designated Contracting States: **AT BE CH DE DK FR GB IT LI NL**

(71) Applicant: **TSUMURA & CO. LTD:**
**12-7, 2-bancho**
**Chyoda-ku, Tokyo 102(JP)**

(72) Inventor: **ISHIDA, Hitoshi**
**200-16, Sena**
**Shizuoka-shi, Shizuoka 420(JP)**
Inventor: **TSUJI, Kuniro**
**1375-11, Ikeda**
**Shizuoka-shi, Shizuoka 422(JP)**
Inventor: **KINOSHITA, Shunya, Tsumura & Co., Ltd.**
**12-7, 2-Bancho, Chiyoda-ku**
**Tokyo 102(JP)**
Inventor: **NAGASAWA, Michio, Tsumura & Co., Ltd.**
**12-7, 2-Bancho, Chiyoda-ku**
**Tokyo 102(JP)**
Inventor: **ABURADA, Masaki, Tsumura & Co., Ltd.**
**12-7, 2-Bancho, Chiyoda-ku**
**Tokyo 102(JP)**
Inventor: **SUEKAWA, Mamoru, Tsumura & Co., Ltd.**
**12-7, 2-Bancho, Chiyoda-ku**
**Tokyo 102(JP)**
Inventor: **ISONO, Masanao, Tsumura & Co., Ltd.**
**12-7, 2-Bancho, Chiyoda-ku**
**Tokyo 102(JP)**

(74) Representative: **Patentanwälte Grünecker, Kinkeldey, Stockmair & Partner**
**Maximilianstrasse 58**
**W-8000 München 22(DE)**

(54) **BILE ALCOHOL AND DRUG CONTAINING THE SAME AS ACTIVE INGREDIENT.**

(57) A novel bile alcohol of general formula (I), having an efficacious physiological activity and being specified in the configurations of particularly the 20-, 24- and 25-positions; formula (I) wherein one of the groups $R_1$ and $R_2$ represents a group of formula (A) or (B) while the other represents -OH or -H; $R_3$ and $R_4$ represent each $-CH_2OH$, $-CH_3$ or $-CH_2OSO_3Na$; or alternatively $R_2$ and $R_3$ are combined together to represent $-OCH_2-$, $R_2$ and $R_4$ are combined together to represent $-OCH_2-$, or $R_3$ and $R_4$ are combined together to represent $-CH_2OCH_2-$; provided that $R_3$ and $R_4$ do not represent $-CH_3$ at the same time and that $R_1$ represents -OH or -H and $R_2$ represents the group of formula (A) or (B) when $R_3$ and $R_4$ represent $-CH_2OH$ at the same time. A drug containing the bile alcohol, including those wherein $R_3$ and $R_4$ in the above formula (I) represent each -H, $-CH_2OH$, $-CH_3$ or $-CH_2OSO_3Na$ (except for the case where both $R_3$ and $R_4$ represent -H at the same time), and 3α 7α, 12α, 24-tetrahydroxy-5α- or -5β-cholane is efficacious as a cerebral function protective agent, cholagogue, cell activator, microcirculation improver, antiarteriosclerotic agent, antihyperlipemia drug, hepatic function

EP 0 489 933 A1

improver, anti-inflammatory drug, antithrombotic drug and hypertensive.

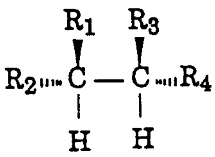 ( I )

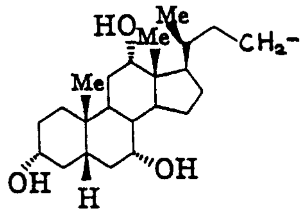 ( A )

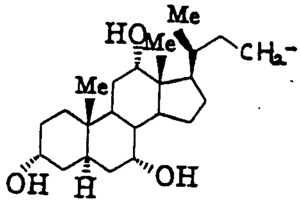 ( B )

## FIELD OF THE INVENTION

This invention relates to a novel bile alcohol having specified configurations and to various drugs such as cerebral function protector, cholagogue, cell activator and the like in which a bile alcohol having useful physiological activities is used as an active ingredient.

## BACKGROUND OF THE INVENTION

It is considered generally that the range of roles of each organ and its secretion products in an animal varies to some extent depending on the degree of evolution. Among bile components which have so far been revealed in the structure, mammal bile acids have been studied since ages ago and, based on the cytological and molecular biological studies, a considerable part of their functions have been revealed in terms of their biosynthetic and metabolic pathways, secretory mechanisms, physiological functions and the like.

For example, it has been revealed that bile alcohols as primary bile acids, such as cholic acid, deoxycholic acid and the like, play important roles in the absorption enhancement of fat-soluble substances, regulation of cholesterol biosynthesis, formation and secretion of bile, and the like functions. In addition, ursodeoxycholic acid as a secondary bile acid whose structure has been revealed based on the studies on bear bile, fel ursi, has already been on the market because of its established functions such as cholagogic effect, fat absorption enhancement, serum cholesterol reduction and the like.

Under such a situation, attempts have been made to obtain derivatives of cholestane and coprostane, and certain cholestane derivatives and coprostane derivatives and their production processes have been disclosed for instance in Biochem. J., vol. 82, p. 285 (1962), Chem. Pharm. Bull., vol. 31 (4), pp. 1330 - 1334 (1983) and Biochem. J., vol. 114, p. 179 (1969). These disclosed compounds, however, are not clear with respect to their configurations, especially at the 24- and 25-positions. As is universally known, differences in such configurations in steroid compounds exert considerable influence especially on their physiological activities.

In addition, JP-A-2-503554 (the term "JP-A" as used herein means an "unexamined published Japanese patent application") and WO89/07607 disclose that a 20S configuration bile alcohol is effective for hepatopathy treatment, intoxication and the like.

Since useful effects as pharmaceutical drugs can be expected from bile alcohols as described in the foregoing, development of a useful bile alcohol having excellent drug effect and specified configurations has been called for in the related areas.

Principal object of this invention, which has been accomplished with the aim of satisfying such a demand, is to provide a novel bile alcohol having specified configurations, as well as various drugs in which a bile alcohol having novel drug effects is used as an active ingredient.

## SUMMARY OF THE INVENTION

The inventors of the present invention have conducted intensive studies and found that a bile alcohol represented by the following general formula (I) has specified novel configurations and is possesed of useful pharmaceutical effects;

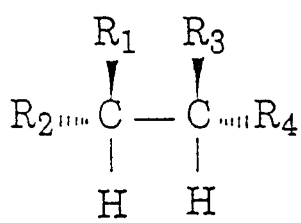

(I)

wherein one of the $R_1$ and $R_2$ is a group represented by the following formula (A) or (B);

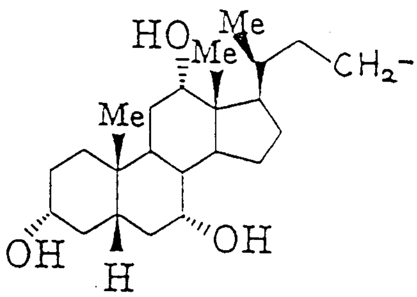

(A)

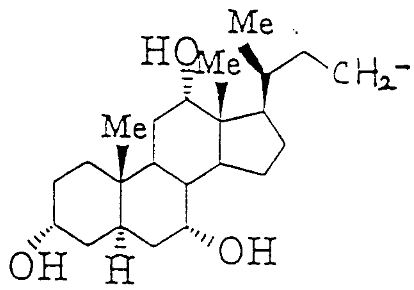

(B)

and the other is -OH or -H, each of $R_3$ and $R_4$ is $-CH_2OH$, $-CH_3$ or $-CH_2OSO_3Na$, in which $R_2$ and $R_3$ may combine with each other to be $-OCH_2-$, $R_2$ and $R_4$ may combine with each other to be $-OCH_2-$ and $R_3$ and $R_4$ may combine with each other to be $-CH_2OCH_2-$, provided that $R_3$ and $R_4$ are not $-CH_3$ at the same time and that $R_1$ is -OH or H and $R_2$ is a group represented by the formula (A) or (B) when $R_3$ and $R_4$ are both $-CH_2OH$ at the same time.

The present inventors have found also that a bile alcohol represented by the following general formula (II) and a bile alcohol represented by the following general formula (III) have various novel and excellent physiological activities and can be used efficiently as a cerebral function protector, a cholagogue, a cell activator, a microcirculation improver, an anti-arteriosclerotic agent, an anti-hyperlipemic agent, a liver function improver, an anti-inflammatory agent, an anti-thrombotic agent and an anti-hypertensive agent;

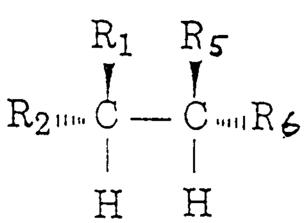

(II)

wherein $R_1$ and $R_2$ are as defined in the foregoing, each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time;

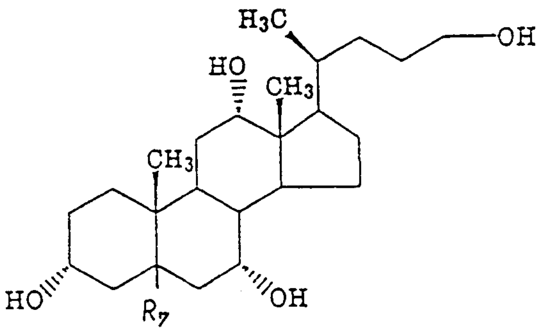

(III)

wherein $R_7$ is α-H or β-H.

## DETAILED DESCRIPTION OF THE INVENTION

As described in the foregoing, the present inventors have conducted intensive studies and found the novel bile alcohol having specified configurations represented by the general formula (I) as a result of the efforts.

While configurations especially at the 24- and 25-positions are undecided or uncertain in the aforementioned prior art cholestane derivatives and coprostane derivatives which have been disclosed for instance in Biochem. J., vol. 82, p.285 (1962), Chem. Pharm. Bull., vol. 31 (4), pp. 1330 - 1334 (1983) and Biochem. J., vol. 114, p. 179 (1969), the bile alcohol of the present invention represented by the general formula (I) has a great significance in terms of its clearly determined configurations, thereby contributing to the expression of its drug effects. In addition, the bile alcohol of the present invention is clearly different from the prior art bile alcohols disclosed in JP-A-2-503554 and WO89/07607, because of a difference in the configuration of the 20-position methyl group and of the specified 25-position configuration.

In the bile alcohol represented by the general formula (I), it is preferable that $R_1$ is a group represented by the formula (A) or (B), $R_2$ is -H or -OH, each of $R_3$ and $R_4$ is $-CH_2OH$, $-CH_3$ or $-CH_2OSO_3Na$, in which $R_2$ and $R_3$ may combine with each other to be $OCH_2$-, $R_2$ and $R_4$ may combine with each other to be $-OCH_2-$ and $R_3$ and $R_4$ may combine with each other to be $-CH_2OCH_2-$. More preferably, $R_1$ is a group represented by the formula (A) or (B), $R_2$ is -H or -OH, and $R_3$ and $R_4$ are different from each other but selected from $-CH_2OH$, $-CH_3$ and $-CH_2OSO_3Na$.

On the other hand, the bile alcohol represented by the general formula (II) is characterized in that its configurations, especially at the 20-, 24- and 25-positions, have successfully been specified and its novel and excellent various drug effects have been found. Novel and excellent drug effects have also been found in 3α,7α,12α,24-tetrahydroxy-5α-cholane and 3α,7α,12α,24-tetrahydroxy-5β-cholane both represented by the general formula (III). In addition, since the bile alcohols according to the present invention are obtained from bile as a secretion product in animals, their toxicities are markedly low and, therefore, they are considerably advantageous with regard to their application to drugs.

According to the present invention, there is provided a pharmaceutical composition which contains, as its active ingredient, the bile alcohol represented by the general formula (II) in which, preferably from its pharmaceutical effect point of view, $R_1$ is a group represented by the formula (A) or (B), $R_2$ is -OH or -H, each of $R_5$ and $R_6$ is $-CH_2OH$ or $-CH_2OSO_3Na$, more preferably, $R_5$ and $R_6$ are both $-CH_2OH$, or $R_5$ is $-CH_2OH$ and $R_6$ is $-CH_2OSO_3Na$.

Each of the aforementioned various pharmaceutical compositions which contain bile alcohols represented by the general formulae (II) and (III) has excellent pharmaceutical effects, especially when the composition is applied to a cerebral function protector, a cell activator, a microcirculation improver, an anti-arteriosclerotic agent, an anti-hyperlipemic agent, an anti-thrombotic agent and an antihypertensive agent.

The bile alcohols of the present invention represented by the general formulae (I), (II) and (III) can be obtained by extracting them from animal bile or by synthesizing them from cholic acid or from a compound obtained during the extraction process.

In the case of the extraction process, bile alcohols are obtained by partitioning animal bile as it is or as its dried form between water and a neutral water-insoluble solvent, subjecting the resulting water layer to partition with a fat-soluble alcohol and then extracting the compounds of interest from the fat-soluble alcohol layer.

Examples of animal bile to be used as the starting material of the extraction process include those of a

shark, a ray, a lamprey, a frog, a sea eel, a sturgeon, a carp and the like.

Examples of the neutral water-insoluble solvent include petroleum ether, ethyl acetate, chloroform and the like, of which ethyl acetate is particularly preferred. Partition is carried out at a temperature within the range of from room temperature to the boiling point of a solvent to be used, thereby removing unnecessary oils and fats such as fatty acids.

The resulting water layer is then subjected to partition using a fat-soluble alcohol, which partition may follow concentrating to an appropriate volume when the volume of the water layer is large.

Illustrative examples of the fat-soluble alcohol include n-butanol, sec-butanol, n-propanol and the like. Partition may be carried out in the usual way, but more efficiently by the addition of salts which include sodium chloride, potassium chloride, ammonium sulfate, sodium sulfate and the like.

It is preferable to use a partition extractor such as Asahina's liquid extractor (mfd. by Kiriyama Seisakusho Co., Ltd.) for the purpose of saving partition-handling trouble.

After completion of the partitioning, bile alcohols of interest are obtained from the fat-soluble alcohol by means of column chromatography, concentration, drying, filtration, recrystallization and the like. Carriers eligible for use in the column chromatography, which may be carried out in the usual way, include silica gel, alumina, HP-20 and the like, and the recrystallization may be effected in the usual way using a solvent or a mixture of two or more solvents selected from the group consisting of water, petroleum ether, n-hexane, n-heptane, benzene, toluene, cyclohexane, chloroform, methylene chloride, ether, tetrahydrofuran, ethyl acetate, acetone, ethanol, methanol and isopropanol.

The above handling steps may be combined in an appropriate order.

In the case of synthetic production, bile alcohols of interest may be obtained from cholic acid or a compound formed during the extraction process as the starting material, by treating the material with at least one reaction step selected from the group consisting of protection of hydroxide groups, reduction, alcoholysis, oxidation, deprotection, hydroboration, tosylation and alkylation.

Protection of a hydroxide group may be effected by using tetrahydropyranyl, methoxymethyl, methoxyethoxymethyl, ethoxyethyl or the like group dissolved in tetrahydrofuran, dimethyl sulfoxide or the like solvent in the presence of a base such as diisopropylamine.

Reduction may be effected for instance by using a reagent such as lithium aluminum hydride, diisobutylaluminum hydride or the like and a solvent such as tetrahydrofuran, ether, dioxane, dimethoxyethane or the like.

Alcoholysis may be carried out in the usual way in which a member of alcohols such as methanol, ethanol and the like is added to the reaction system in the presence of a catalyst such as sulfuric acid, toluenesulfonic acid, camphorsulfonic acid or the like.

Oxidation may be effected for example by a process in which pyridinium dichromate, pyridinium chloromate, pyridinium chloromate-on-alumina or the like is used as a reagent and methylene chloride or the like is used as a solvent, or by a process in which the reaction is carried out using hydrogen peroxide in an alkaline aqueous solution.

Hydroboration may be carried out according to the usually used means in which diborane, borane-tetrahydrofuran complex, borane-dimethyl sulfide complex or the like is used, and tetrahydrofuran or the like as a solvent.

Tosylation may be effected by usually used means using tosyl chloride as a reagent and pyridine or the like as a solvent.

Alkylation may be effected making use of for instance a process in which an alkyl acrylate such as methyl acrylate, ethyl acrylate or the like is used, or a process in which diethyl malonate is used together with dimethylformamide or the like as a solvent.

Deprotection is generally carried out by acid or alkali hydrolysis, but such a process may not be suitable for the production of the compounds of the present invention because of the generation of undesirable side reactions. In consequence, deprotection may preferably be effected by allowing the material to contact with a weakly acidic cation resin. A preferred illustrative example of such a weakly acidic cation resin is Dowex 50W-X8. This process can also be used for the elimination of a sulfuric ester group from the aforementioned compound extracted from animal bile.

The thus obtained compounds of the present invention may, if necessary, be purified by means of recrystallization, silica gel-aided column chromatography and the like.

Next, dose and pharmaceutical preparation of the compounds of the present invention are described.

Each of the compounds of the present invention can be administered to animals and human as it is or together with usually used pharmaceutical carriers. Dosage form is not strictly limited and therefore can be selected as occasion calls from oral administration forms such as tablets, capsules, granules, fine subtilaes, powders and the like, and non-oral administration forms such as injections, suppositories and the like.

Dose of the compound may vary depending on the age, weight, state of a disease and the like of each patient to be treated, but it is preferable to administer, when administered orally to an adult, in an amount of from 10 mg to 6 g per day as the amount of the compound of the present invention by dividing the necessary amount into several times.

Oral drugs may be prepared in the usual way by mixing the compound of the present invention with fillers such as starch, lactose, sucrose, mannitol, carboxymethyl cellulose, corn starch, inorganic salts and the like.

Such type of pharmaceutical preparations may further contain, in addition to the above fillers, a binder, a disintegrating agent, a surface active agent, a lubricant, a fluidity enhancer, a corrective agent, a coloring agent, an aromatic agent and the like. The following illustrates examples of these auxiliary components.

[Binders]

Illustrative examples of binders include starch, dextrin, gum arabic in powder form, gelatin, hydroxypropyl starch, methyl cellulose, sodium carboxymethyl cellulose, hydroxypropyl cellulose, crystalline cellulose, ethyl cellulose, polyvinyl pyrrolidone, macrogol and the like.

[Disintegrating agents]

Illustrative examples of Disintegrating agents include starch, hydroxypropyl starch, sodium carboxymethyl cellulose, calcium carboxymethyl cellulose, carboxymethyl cellulose, low-substituted hydroxypropyl cellulose and the like.

[Surface active agents]

Illustrative examples of surface active agents include sodium lauryl sulfate, soy bean lecithin, sucrose-fatty acid esters, polysorbate 80 and the like.

[Lubricants]

Illustrative examples of lubricants include talc, waxy materials, hydrogenated plant oil, sucrose-fatty acid esters, magnesium stearate, calcium stearate, aluminium stearate, polyethylene glycol and the like.

[Fluidity enhancers]

Illustrative examples of fluidity enhancers include light silicic anhydride, dried aluminium hydroxide gel, synthetic aluminium silicate, magnesium silicate and the like.

The compound of the present invention may also be administered as suspensions, emulsions, medicated syrups or elixirs, which may contain a corrective agent, a coloring agent and the like.

When the compound is used as non-oral administration drugs, it is preferable to administer the compound by intravenous injection, intravenous drip infusion, subcutaneous injection or intramuscular injection in an amount of from 0.5 mg to 200 mg per day per adult as the amount of the compound of the present invention, though dose of the compound may vary depending on the age, weight, state of a disease and the like of each patient to be treated.

Such non-oral administration compositions may be prepared by usually used means using an appropriate diluent which is generally selected from distilled water for injection use, physiological saline, aqueous glucose solution, plant oil for injection use, sesame oil, peanut oil, soybean oil, corn oil, propylene glycol, polyethylene glycol and the like. If necessary, bacteriocides, antiseptics, stabilizers and the like may be further added. From a stability point of view, the non-oral administration preparation may be filled in vials and the like, frozen and then freeze-dried in the usual way to remove moisture, so that a solution can be prepared from the freeze-dried preparation at the time of using. If necessary, it may be mixed further with a tonicity agent, a stabilizer, an antiseptic agent, a soothing agent and the like.

Other non-oral administration forms include coating preparations such as liquids for external use and ointments, suppositories for rectal administration use and the like which can be prepared by usually used means.

The following describes preferred examples for the production of the compounds of the present invention.

EXAMPLE 1

Freeze-dried powder of flathead shark bile (11.01 g) was dissolved in water, unnecessary substances were removed by chloroform extraction, the resulting water layer was saturated with sodium chloride and then extraction was carried out with n-butanol. The thus obtained n-butanol extract (about 5.82 g; contains salts) was applied to silica gel column chromatography and eluted using lower layer of chloroform/methanol/water system (65:35:10) to obtain 2.94 g of (24R)-(25S)-(+)-3α,7α,12α, 24,26-pentahydroxy-5β-cholestane-27-yl sodium sulfate.

(24R)-(25S)-(+)-3α,7α,12α,24,26-pentahydroxy-5β-cholestane-27-yl sodium sulfate:
white powder; sol in pyridine, $H_2O$, EtOH; insol in acetone, AcOEt, $CHCl_3$
$[\alpha]_D^{22}$ = 21.8 (0.5c in MeOH)

| Anal. Calcd. for $C_{27}H_{47}O_9SNa$: | | | | |
|---|---|---|---|---|
| | C; 56.82, | H; 8.30, | S; 5.62, | Na; 4.03 |
| Found: | C; 56.99, | H; 8.79, | S; 5.62, | Na; 4.23 |

SIMS mass (m/e):
654 $[C_{27}H_{47}SO_9 \cdot HN(C_2H_6O)_2]$,
574 $[C_{27}H_{47}O_6 \cdot HN(C_2H_6O)_2]$

IR $\nu_{max}^{KBr}$ $cm^{-1}$:

3420, 2950, 1470, 1380, 1230, 1070, 980, 910, 810
$^1$H-NMR (in $CD_3OD$) δ(ppm):
4.22 (1H, dd, J=4.5, 10.0Hz), 4.11 (1H, dd, J=6.7, 10.0Hz), 4.00 (1H, br), 3.80 (1H, m), 3.80-3.62 (3H, m), 3.45-3.30 (1H, m), 2.35-2.15 (2H, m), 2.05-1.02 (23H, m), 1.02 (3H, d, J=6, 25Hz), 0.92 (3H, s), 0.72 (3H, s)
$^{13}$C-NMR (in $CD_3OD$) δ(ppm):
74.1(d), 72.9(d), 71.4(d), 69.1(d), 66.7(t), 61.2(t), 48.3(d), 47.8, 47.5, 43.1(d), 43.0(s), 41.0(d), 40.3(d), 37.0(d), 36.5(t), 35.9(d), 35.8(t), 33.3(s), 32.1(t), 31.2(t), 29.5(t), 28.8(t), 27.9(d), 24.3(t), 23.2(q), 18.1(q), 13.1(q)

EXAMPLE 2

Freeze-dried powder of salmon shark bile (12.38 g) was dissolved in water, unnecessary substances were removed by chloroform extraction, the resulting water layer was saturated with sodium chloride and then extraction was carried out with n-butanol. The thus obtained n-butanol extract (Ca. 5.3 g; contains salts) was applied to silica gel column chromatography and eluted using lower layer of chloroform/methanol/water system (65:35:10) to obtain a mixed fraction (1.89 g) of (24R)-(25S)-(+)-3α,7α,12α,24,26-pentahydroxy-5β-cholestane-27-yl sodium sulfate and (24R)-(25R)-(+)3α,7α,12α,24,26-pentahydroxy-5β-cholestane-27-yl sodium sulfate. The thus obtained fraction of 160.4 mg was subjected to purification using a high performance liquid silica gel column chromatography (Column, YMC-Pack A-324 (ODS) 10×300 mm) to obtain 29.3 mg of (24R)-(25S)-(+)-3α,7α,12α,24,26-pentahydroxy-5β-cholestane-27-yl sodium sulfate and 18.1 mg of (24R)-(25R)-(+)-3α,7α,12α,24,26-pentahydroxy-5β-cholestane-27-yl sodium sulfate.

(24R)-(25R)-(+)-3α,7α,12α,24,26-pentahydroxy-5β-cholestane-27-yl sodium sulfate:
white powder; sol in $H_2O$, MeOH, EtOH, pyridine; insol in acetone, AcOEt, $CHCl_3$
$[\alpha]_D^{18}$ = 33.8 (0.5c in MeOH)

| Anal. Calcd. for $C_{27}H_{47}O_9SNa$: | | | | |
|---|---|---|---|---|
| | C; 56.82, | H; 8.30, | S; 5.62, | Na; 4.03 |
| Found: | C; 56.51, | H; 8.73, | S; 5.55, | Na; 3.79 |

SIMS mass (m/e):
759 $[C_{27}H_{47}O_9S \cdot 2HN(C_2H_6O)_2]$,
654 $[C_{27}H_{47}O_9S \cdot HN(C_2H_6O)_2]$,
574 $[C_{27}H_{47}O_6 \cdot HN(C_2H_6O)_2]$

$$IR\ \nu_{max}^{KBr}\ cm^{-1}:$$

3420, 2950, 1470, 1380, 1230, 1070, 980, 910, 810

$^{1}H$-NMR (in $D_2O$) δ(ppm):

4.18 (1H, dd, J=5.8, 10.1Hz), 4.14 (1H, dd, J=5.9, 10.0Hz), 4.06 (1H, br), 3.89 (1H, br), 3.82 (1H, dd, J=4.7, 11.5Hz), 3.77 (1H, br), 3.71 (1H, dd, J=6.7, 11.5Hz), 3.49 (1H, br), 2.20-1.24 (29H, m), 1.02 (3H, d, J=6, 1Hz), 0.92 (3H, s), 0.71 (3H, s)

$^{13}C$-NMR (in $D_2O$) δ(ppm):

75.8(d), 74.3(d), 73.1(d), 70.9(d), 69.7(t), 61.7(t), 49.4(d), 49.0(s), 48.3(d), 44.3(d), 44.1(d), 42.4(d), 41.2(t), 38.6(d), 37.9(t), 37.3(s), 36.9(t), 34.5(t), 32.9(t), 32.0(t), 30.7(t), 30.3(t), 29.2(d), 26.0(t), 25.2(q), 19.9(q), 15.2-(q)

EXAMPLE 3

A 6.0 g portion of (24R)-(25S)-(+)-3α,7α,12α,24,26-pentahydroxy-5β-cholestane-27-yl sodium sulfate obtained in Example 1 was dissolved in 60 ml of 2.5 N potassium hydroxide and the solution was heated for 16 hours under reflux to complete the reaction. After spontaneous cooling, the resulting reaction solution was mixed with 100 ml of water and then extracted three times with 150 ml of ethyl acetate/n-butanol system (1:1) to obtain 5.0 g of crude reaction product. The thus extracted crude product was dissolved in the lower layer of chloroform/methanol/ water system (70:30:10), applied to dry-packed silica gel column chromatography and then eluted using the same solvent system followed by subjecting to thin-layer silica gel column chromatography. Eluted fractions from 180 to 1005 ml were pooled and solvents in the thus collected pool were removed under a reduced pressure to obtain 3.1 g of crude fraction. Thereafter, the crude fraction was recrystallized from ethanol/ethyl acetate (m.p. 120-121 °C) or ethanol/water (m.p. 124-126 °C), acetone (m.p., 196-197 °C) to obtain 2.40 g of colorless plate crystals of (24R)-(25S)-(+)-24,26-epoxy-5β-cholestane-3α,7α,12α,27-tetrol.

(24R)-(25S)-(+)-24,26-epoxy-5β-cholestane-3α,7α,12α,27-tetrol:

m.p. 196-197 °C (189-190 °C)

colorless plate,

insol in $H_2O$, sol in MeOH, EtOH, pyridine, slightly sol in AcOEt, $CHCl_3$

$[\alpha]_D^{20}$ = 39.6 (1.0c in MeOH)

| Anal. Calcd. for $C_{27}H_{46}O_5$: | | |
|---|---|---|
| | C; 71.96, | H; 10.29 |
| Found: | C; 71.83, | H; 10.23 |

| High resolution mass: | | |
|---|---|---|
| Calcd. for $C_{27}H_{44}O_4$ (des-$H_2O$): | 432, | 324 |
| Found | 432, | 326 |

$$IR\ \nu_{max}^{KBr}\ cm^{-1}:$$

3450, 2950, 2875, 1480, 1380, 1080, 1040, 1020, 980, 955, 920, 860

$^{1}H$-NMR (in Acetone-$d_6$) δ(ppm):

4.45 (1H, dd, J=5.9, 8.1Hz), 4.41 (1H, dt, J=6.2, 6.2Hz), 4.28 (1H, dd, J=6.2, 6.2Hz), 3.98-3.96 (1H, m), 3.81-3.80 (1H, m), 3.76 (1H, dd, J=4.4, 6.0Hz), 3.72 (1H, dd, J=4.5, 6.2Hz), 3.71 (1H, m), 3.37-3.29 (2H, m), 3.27 (1H, d, J=4.2Hz), 3.06 (1H, d, J=3.32Hz), 2.72 (1H, 5d, J=6.2, 6.2, 6.2, 6.2, 7.9Hz), 2.40-2.29 (2H, m), 2.21-2.13 (1H, m), 1.97-1.91 (2H, m), 1.86-1.72 (4H, m), 1.62-1.48 (7H, m), 1.44-1.21 (6H, m), 1.15-1.06 (2H, m), 1.03 (3H, d, J=6.6Hz), 0.96 (1H, dd, J=3.5, 14.1Hz), 0.91 (3H, s), 0.71 (3H, s)

$^{13}C$-NMR (in Acetone-$d_6$) δ(ppm):

85.9(d), 73.2(d), 72.4(d), 70.5(t), 68.4(d), 64.1(t), 47.8(d), 47.3(s), 44.2(d), 43.1(d), 42.8(d), 41.1(d), 40.9(t), 36.5(d), 36.5(t), 36.0(t), 35.7(s), 34.6(t), 31.6(t), 31.1(t), 29.5(t), 28.6(t), 27.7(d), 24.0(d), 23.4(q), 18.2(q), 13.1-(q)

Crystal data of (24R)-(25S)-(+)-24,26-epoxy-5$\beta$-cholestane-3$\alpha$,7$\alpha$,12$\alpha$,27-tetrol

$C_{27}H_{46}O_5$, M = 450.66, Orthorhombic, Space group P $2_1$ $2_1$ $2_1$

a = 13.562(2), b = 21.636(2), c = 8.735(2)Å, V = 2563.1Å$^3$

Z = 4, F(000) = 992, Dcalcd. = 1.17 g/cm$^3$,

Dobsd. = 1.16 g/cm$^3$

$\lambda$(Mo-$K_\alpha$) = 0.71070Å, $\mu$(Mo-$K_\alpha$) = 0.7 cm$^{-1}$,

crystal size 0.4×0.4×0.2 mm

Positional parameters and thermal parameters with their estimated standard deviations in parentheses denoting the least significant digits

| NO | ATOM | $X\times10^4$ | $Y\times10^4$ | $Z\times10^4$ | B11 | B22 | B33 | B12 | B13 | B23 |
|---|---|---|---|---|---|---|---|---|---|---|
| 1 | 01 | 8033(4) | 4457(3) | 204(7) | 63(3) | 34(2) | 225(10) | -20(4) | -23(7) | -62(11) |
| 2 | 02 | 8881(3) | 5905(3) | 4292(6) | 48(3) | 27(1) | 148(8) | -5(3) | -8(5) | 33(8) |
| 3 | 03 | 11982(3) | 5635(2) | 2379(5) | 46(3) | 21(1) | 138(7) | 3(3) | -5(5) | 16(7) |
| 4 | 04 | 15633(4) | 5884(3) | 7734(8) | 46(3) | 45(2) | 78(13) | -4(4) | -22(9) | 13(11) |
| 5 | 05 | 13144(4) | 5101(2) | 10074(7) | 63(3) | 34(2) | 206(9) | -13(4) | -40(6) | 64(10) |
| 6 | C6 | 9301(6) | 5875(4) | -1194(9) | 57(5) | 40(3) | 119(11) | -8(6) | 28(9) | -25(12) |
| 7 | C7 | 9181(5) | 5205(4) | -851(9) | 45(4) | 45(3) | 140(11) | -3(5) | -14(10) | -7(12) |
| 8 | C8 | 8179(5) | 5079(4) | -132(9) | 46(4) | 37(3) | 165(12) | -9(5) | 3(9) | -35(13) |
| 9 | C9 | 8039(5) | 5492(4) | 1262(9) | 36(3) | 35(2) | 146(11) | 3(5) | -8(8) | -5(11) |
| 10 | C10 | 8183(5) | 6158(4) | 955(9) | 34(3) | 39(3) | 185(13) | 15(5) | 32(9) | -27(12) |
| 11 | C11 | 8006(5) | 6541(4) | 2406(10) | 37(3) | 30(2) | 232(15) | 18(5) | 18(10) | 31(13) |
| 12 | C12 | 8842(5) | 6493(4) | 3561(9) | 45(4) | 30(2) | 163(12) | 11(5) | -13(9) | 37(11) |
| 13 | C13 | 9843(4) | 6634(3) | 2828(8) | 39(3) | 21(2) | 164(12) | 11(4) | 14(8) | 20(10) |
| 14 | C14 | 10021(4) | 6230(3) | 1399(8) | 37(3) | 24(2) | 124(9) | 2(4) | 25(7) | 11(9) |
| 15 | C15 | 9187(5) | 6313(4) | 177(8) | 41(3) | 43(3) | 141(11) | 10(5) | 59(10) | -14(11) |

| | | | | | | | | | | |
|---|---|---|---|---|---|---|---|---|---|---|
| 16 | C16 | 11061(4) | 6337(4) | 734(8) | 37(3) | 37(2) | 124(10) | -2(5) | 15(9) | 12(10) |
| 17 | C17 | 11881(4) | 6266(3) | 1916(7) | 36(3) | 22(2) | 138(10) | -5(4) | 24(7) | 30(10) |
| 18 | C18 | 11709(4) | 6681(3) | 3310(7) | 43(3) | 19(1) | 117(9) | -6(4) | 2(6) | 16(9) |
| 19 | C19 | 10685(4) | 6530(3) | 3958(7) | 46(3) | 17(1) | 118(9) | -2(4) | 16(6) | 4(9) |
| 20 | C20 | 10649(5) | 6845(4) | 5493(9) | 55(4) | 37(3) | 136(11) | -8(5) | -20(9) | 20(12) |
| 21 | C21 | 11724(6) | 6792(4) | 6095(10) | 65(5) | 36(3) | 167(13) | -3(6) | -24(9) | 2(14) |
| 22 | C22 | 12354(5) | 6541(3) | 4743(8) | 60(4) | 16(1) | 154(11) | -8(4) | -12(7) | -9(11) |
| 23 | C23 | 9183(8) | 6987(5) | -424(12) | 74(6) | 40(3) | 221(17) | 13(7) | 78(11) | -23(19) |
| 24 | C24 | 11795(6) | 7368(4) | 2854(11) | 58(5) | 27(2) | 220(15) | -5(5) | 34(9) | 27(15) |
| 25 | C25 | 13435(5) | 6784(3) | 4829(9) | 49(4) | 26(2) | 193(13) | -10(4) | -17(8) | -21(13) |
| 26 | C26 | 14037(6) | 6598(4) | 3429(12) | 42(4) | 32(2) | 255(16) | -11(5) | -37(11) | 12(14) |
| 27 | C27 | 13920(6) | 6567(4) | 6293(11) | 625(5) | 24(2) | 221(14) | -11(5) | -12(9) | -51(14) |
| 28 | C28 | 13975(7) | 5878(4) | 6425(11) | 78(5) | 32(2) | 190(14) | -15(6) | -28(10) | -53(16) |
| 29 | C29 | 14637(5) | 5616(4) | 7682(9) | 47(4) | 25(2) | 200(13) | 9(4) | -2(9) | 23(12) |
| 30 | C30 | 14475(5) | 5799(3) | 9370(10) | 47(4) | 22(2) | 206(13) | 2(4) | -18(9) | 14(13) |
| 31 | C31 | 15576(6) | 5946(6) | 9341(14) | 64(5) | 43(3) | 299(24) | -28(7) | -41(15) | -91(19) |
| 32 | C32 | 14162(6) | 5277(4) | 10379(10) | 69(5) | 32(2) | 165(13) | 4(6) | -28(9) | -2(15) |

| NO | ATOM | $X\times10^3$ | $Y\times10^3$ | $Z\times10^3$ | BEQA··2 |
|---|---|---|---|---|---|
| 33 | H1 | 820(8) | 436(5) | 108(12) | 6.01 |
| 34 | H2 | 888(6) | 625(5) | 485(11) | 4.42 |
| 35 | H3 | 1222(6) | 549(4) | 156(11) | 3.87 |
| 36 | H5 | 1184(6) | 515(4) | 406(11) | 5.77 |
| 37 | H6A | 884(7) | 599(4) | -200(11) | 5.13 |
| 38 | H6B | 998(7) | 599(4) | -162(11) | 5.13 |
| 39 | H7A | 927(6) | 494(4) | -183(11) | 5.35 |
| 40 | H7B | 969(6) | 499(4) | -11(11) | 5.35 |
| 41 | H8 | 775(6) | 521(4) | -101(10) | 5.16 |
| 42 | H9A | 737(6) | 549(4) | 181(10) | 4.55 |
| 43 | H9B | 854(6) | 532(4) | 212(10) | 4.55 |
| 44 | H10 | 765(6) | 627(4) | 23(11) | 5.17 |
| 45 | H11A | 802(6) | 697(4) | 197(10) | 5.17 |
| 46 | H11B | 737(7) | 637(4) | 291(10) | 5.17 |
| 47 | H12 | 868(7) | 671(4) | 428(11) | 4.64 |
| 48 | H13 | 980(6) | 707(4) | 243(10) | 3.95 |
| 49 | H14 | 1001(6) | 580(4) | 176(9) | 3.70 |

| | | | | | |
|---|---|---|---|---|---|
| 50 | H16A | 1117(6) | 596(4) | -10(10) | 4.53 |
| 51 | H16B | 1102(6) | 683(4) | 25(10) | 4.53 |
| 52 | H17 | 1243(6) | 642(4) | 142(9) | 3.66 |
| 53 | H19 | 1070(6) | 614(4) | 420(9) | 3.37 |
| 54 | H20A | 1038(6) | 742(4) | 534(11) | 5.06 |
| 55 | H20B | 1020(6) | 656(4) | 613(11) | 5.06 |
| 56 | H21A | 1191(7) | 725(4) | 640(11) | 5.54 |
| 57 | H21B | 1173(7) | 653(4) | 711(11) | 5.54 |
| 58 | H22 | 1235(5) | 613(4) | 483(10) | 4.03 |
| 59 | H23A | 979(8) | 706(5) | -92(13) | 6.55 |
| 60 | H23B | 862(8) | 703(5) | -110(12) | 6.55 |
| 61 | H23C | 916(8) | 716(5) | 42(13) | 6.55 |
| 62 | H24A | 1125(7) | 755(4) | 215(11) | 5.34 |
| 63 | H24B | 1173(7) | 758(4) | 391(11) | 5.34 |
| 64 | H24C | 1251(7) | 752(4) | 253(10) | 5.34 |
| 65 | H25 | 1339(6) | 737(4) | 498(10) | 4.78 |
| 66 | H26A | 1394(7) | 616(5) | 333(11) | 5.64 |
| 67 | H26B | 1378(7) | 685(4) | 226(12) | 5.64 |

| | | | | | |
|---|---|---|---|---|---|
| 68 | H26C | 1469(7) | 665(4) | 352(11) | 5.64 |
| 69 | H27A | 1464(7) | 662(4) | 630(11) | 5.31 |
| 70 | H27B | 1351(7) | 670(4) | 728(11) | 5.31 |
| 71 | H28A | 1321(7) | 560(4) | 687(11) | 5.90 |
| 72 | H28B | 1422(7) | 563(4) | 535(11) | 5.90 |
| 73 | H29 | 1472(6) | 512(4) | 748(10) | 4.76 |
| 74 | H30 | 1405(6) | 607(4) | 949(11) | 4.67 |
| 75 | H31A | 1595(8) | 574(5) | 1009(14) | 7.35 |
| 76 | H31B | 1565(7) | 637(5) | 951(13) | 7.35 |
| 77 | H32A | 1461(6) | 482(4) | 1010(11) | 5.37 |
| 78 | H32B | 1420(7) | 545(4) | 1148(11) | 5.37 |

EXAMPLE 4

Freeze-dried powder of salmon shark bile (12.38 g) was dissolved in water, unnecessary substances were removed by chloroform extraction, the resulting water layer was saturated with sodium chloride and then extraction was carried out with n-butanol. The thus obtained n-butanol extract (Ca. 5.3 g; contains salts) was applied to silica gel column chromatography and eluted using lower layer of chloroform/methanol/water system (65:35:10) to obtain a mixed fraction (1.89 g) of (24R)-(25S)-(+)-3α,7α,12α,24,26-pentahydroxy-5β-cholestane-27-yl sodium sulfate and (24R)-(25R)-(+)-3α,7α,12α,24,26-pentahydroxy-5β-cholestane-27-yl sodium sulfate.

A 160.4 mg portion of the thus obtained fraction was subjected to purification using a high performance liquid column chromatography (Column, YMC-Pack A-324 (ODS) 10 × 300 mm) to obtain 18.1 mg of (24R)-(25R)-(+)-3α,7α,12α,24,26-pentahydroxy-5β-cholestane-27-yl sodium sulfate.

Next, 5 mg of (24R)-(25R)-(+)-3α,7α,12α,24,26-pentahydroxy-5β-cholestane-27-yl sodium sulfate thus obtained was dissolved in 2 ml of 2.5 N potassium hydroxide and the solution was heated at 115°C for 16 hours under reflux to complete the reaction. After spontaneous cooling, the resulting reaction solution was mixed with 10 ml of water and then extracted three times with 20 ml of ethyl acetate. After washing three times with 30 ml of water, solvent was removed under a reduced pressure. Thereafter, the thus obtained residue was subjected to purification using a high performance liquid column chromatography (Column, YMC-Pack A-324 (ODS) 10×300 mm) to obtain 3.9 mg of (24R)-(25R)-(+)-24,26-epoxy-5β-cholestane-3α,7α,12α,24-tetrol and 4.0 mg of (24R)-(+)-26,27-epoxy-5β-cholestane-3α,7α,12α,24-tetrol.

(24R)-(25R)-(+)-24,26-epoxy-5β-cholestane-3α,7α,12α,27-tetrol:

m.p. 194-195°C (colorless needle)

insol in $H_2O$, sol in MeOH, EtOH, pyridine, slightly sol in AcOEt, $CHCl_3$

$[\alpha]_D^{18}$ = 27.7 (0.7c in MeOH)

| Anal. Calcd. for $C_{27}H_{46}O_5$: | | |
| --- | --- | --- |
| | C; 71.96, | H; 10.29 |
| Found: | C; 70.65, | H; 10.15 |

| High resolution mass: | | |
| --- | --- | --- |
| Calcd. for $C_{27}H_{44}O_4$ (des-$H_2O$): | 432, | 324 |
| Found | 432, | 326 |

IR $\nu_{max}^{KBr}$ $cm^{-1}$:

3450, 2950, 2875, 1480, 1380, 1080, 1040, 1020, 980, 955, 920, 860

$^1$H-NMR (in Acetone-$d_6$) δ(ppm):

4.71 (1H, ddd, J=5.1, 7.6, 8.7Hz), 4.57 (1H, dd, J=6.0, 7.6Hz), 4.11 (1H, dd, J=5.7, 5.7Hz), 3.97 (1H, m), 3.92 (1H, ddd, J=4.9, 7.4, 10.9Hz), 3.81 (1H, m), 3.79 (1H, ddd, J=4.7, 7.6, 10.9Hz), 3.63 (1H, t, J=4.9Hz), 3.37-3.29 (2H, m), 3.26 (1H, d, J=4.2Hz), 3.07 (1H, d, J=3.3Hz), 3.02 (1H, 5d, J=5.4, 7.5, 7.5, 7.5, 7.5Hz), 2.40-2.29 (2H, m), 2.16 (1H, ddd, J=12.6, 12.6, 7.5Hz), 1.97-1.90 (2H, m), 1.87-1.69 (4H, m), 1.63-1.48 (8H, m), 1.43-1.19 (6H, m), 1.16-1.05 (2H, m), 1.03-1.01 (3H, d, J=6.6Hz), 0.98-0.90 (1H, dd, J=14.1, 3.5Hz), 0.90 (3H, s), 0.71 (3H, s)

$^{13}$C-NMR (in Acetone-$d_6$) δ(ppm):

84.6(d), 73.2(d), 72.4(d), 71.4(t), 68.4(d), 61.5(t), 47.8(d), 47.3(s), 43.1(d), 42.8(d), 41.1(d), 40.9(t), 40.7(d), 36.6(d), 36.6(t), 36.0(t), 35.7(t), 32.0(t), 31.6(t), 29.8(t), 29.4(t), 28.6(t), 27.7(d), 24.1(t), 23.4(q), 18.1(q), 13.1-(q)

EXAMPLE 5

Freeze-dried powder of salmon shark bile (12.38 g) was dissolved in water, unnecessary substances were removed by chloroform extraction, the resulting water layer was saturated with sodium chloride and then extraction was carried out with n-butanol. The thus obtained n-butanol extract (Ca. 5.3 g; contains salts) was applied to silica gel column chromatography and eluted using lower layer of chloroform/methanol/water system (65:35:10) to obtain a mixed fraction (1.89 g) of (24R)-(25S)-(+)-3α,7α,12α,24,26-pentahydroxy-5β-cholestane-27-yl sodium sulfate and (24R)-(25R)-(+)-3α,7α,12α,24,26-pentahydroxy-5β-cholestane-27-yl sodium sulfate.

A 160.4 mg portion of the thus obtained fraction was subjected to purification using a high performance liquid column chromatography (Column, YMC-Pack A-324 (ODS) 10×300 mm) to obtain 18.1 mg of (24R)-(25R)-(+)-3α,7α,12α,24,26-pentahydroxy-5β-cholestane-27-yl sodium sulfate.

Next, 5 mg of (24R)-(25R)-(+)-3α,7α,12α,24,26-pentahydroxy-5β-cholestane-27-yl sodium sulfate thus obtained was dissolved in 2 ml of 2.5 N potassium hydroxide and the solution was heated at 115°C for 16 hours under reflux to complete the reaction. After spontaneous cooling, the resulting reaction solution was mixed with 10 ml of water and then extracted three times with 20 ml of ethyl acetate. After washing three times with 30 ml of water, solvent was removed under a reduced pressure. Thereafter, the thus obtained residue was subjected to purification using a high performance liquid column chromatography (Column, YMC-Pack A-324 (ODS) 10×300 mm) to obtain 3.9 mg of (24R)-(25R)-(+)-24,26-epoxy-5β-cholestane-3α,7α,12α,27-tetrol and 4.0 mg of (24R)-(+)-26,27-epoxy-5β-cholestane-3α,7α,12α,24-tetrol.

(24R)-(+)-26,27-epoxy-5β-cholestane-3α,7α,12α,24-tetrol:

m.p. 199-200°C (colorless needle)

insol in $H_2O$, sol in MeOH, EtOH, pyridine, slightly sol in AcOEt, $CHCl_3$

$[\alpha]_D^{18}$ = 28.9 (0.6c in MeOH)

| Anal. Calcd. for $C_{27}H_{46}O_5$: | | |
|---|---|---|
| | C; 71.96, | H; 10.29 |
| Found: | C; 70.26, | H; 10.10 |

| High resolution mass: | | |
|---|---|---|
| Calcd. for $C_{27}H_{44}O_4$ (des-$H_2O$): | 432, | 324 |
| Found | 432, | 324 |

$$IR\ \nu_{max}^{KBr}\ cm^{-1}:$$

3450, 2950, 2875, 1640, 1470, 1380, 1080, 1040, 1010, 980, 950, 920, 860

$^1$H-NMR (in pyridine-$d_6$) $\delta$(ppm):

6.22 (1H, br), 5.70 (1H, br), 5.36 (1H, br), 5.30 (1H, br), 5.01 (1H, dd, J=6.1, 6.1Hz), 4.88 (1H, dd, J=5.7, 7.9Hz), 4.78 (1H, dd, J=5.8, 8.1Hz), 4.70 (1H, dd, J=6.1, 6.1Hz), 4.28 (1H, br), 4.10 (1H, br), 4.05 (1H, br), 3.75 (1H, br), 3.20-3.07 (2H, m), 2.92 (1H, dt, J=4.0, 12.5Hz), 2.75 (1H, dt, 6.9, 12.3Hz), 2.37 (1H, q, J=9.6Hz), 2.14-1.96 (3H, m), 1.96-1.76 (6H, m) , 1.72-1.30 (9H, m), 1.27-1.18 (1H, m), 1.24 (3H, d, J=6.2Hz), 1.07 (1H, dt, J=4.2, 13.4Hz), 1.01 (3H, s), 0.84 (3H, s)

$^{13}$C-NMR (in Pyridine-$d_5$) $\delta$(ppm):

74.7(t), 73.9(t), 72.6(d), 72.5(d), 71.9(d), 67.7(d), 47.4(s), 46.9(d), 42.7(d), 42.6(d), 41.9(d), 41.0(t), 40.7(d), 36.3(t), 36.2(d), 35.9(t), 35.4(s), 32.3(t), 32.0(t), 31.7(t), 29.7(t), 28.3(t), 27.4(d), 23.8(t), 23.2(q), 17.9(q), 13.1-(q)

EXAMPLE 6

Methyl 3α,7α,12α-trihydroxy-5β-cholan-24-oate (10.0 g; 23.7 mM) obtained by the method of R.R. Moffett et al. and 43 mg of p-toluene sulfonic acid monohydrate as a catalyst are dissolved in 10 ml of methylene chloride, and a methylene chloride solution (100 ml) containing 3,4-dihydro-2H-pyran (41.5 ml, 45.05 g; 535 mM) is added dropwise to the former solution with stirring under water-cooling condition spending 30 minutes. After completion of the dropwise addition, the thus prepared mixture is stirred for 5 hours at room temperature and the resulting reaction solution is diluted with 400 ml of diethyl ether. Thus extracted solution is washed with 150 ml of 5% aqueous solution of sodium hydrogencarbonate, 100 ml of purified water and 100 ml of saturated aqueous solution of sodium chloride and then dried using anhydrous magnesium sulfate. After removing solvent by distillation under a reduced pressure, the thus obtained reaction product was purified by silica gel column chromatography using n-hexane/ethyl acetate (3:1) as the elution solvent. Thus obtained product, methyl 3α,7α,12α-tritetrahydropyranoxy-5β-cholan-24-oate, is pale yellow and oily. (yield; 15.03 g, 94.13%)

Methyl 3α,7α,12α-tritetrahydropyranoxy-5β-cholan-24-oate:

$$IR\ \nu_{max}^{KBr}\ cm^{-1}:$$

3470, 2934, 2866, 1737, 1438, 1024, 985

$^1$H-NMR (in $CDCl_3$) $\delta$(ppm):

4.81 - 4.55 (3H, m), 4.06 - 3.25 (9H, m), 3.66 (3H, s), 2.44 - 1.11 (41H, m), 1.02, 1.00 (3H, d, J=4.95Hz), 0.91, 0.89 (4H, d, J=2.97Hz), 0.70, 0.69, 0.66, 0.66 (3H, dd, J=1.98Hz, 2.31Hz)

$^{13}$C-NMR (in $CDCl_3$) $\delta$(ppm):

174.58, 174.48 (S, C-24), 100.75, 100.56, 98.50, 98.41, 98.17, 98.04, 96.76, 96.50, 96.45, 96.37, 96.10, 95.00, 94.87, 94.79, 94.74, 94.31, 91.52, 91.37, 81.88, 81.67, 81.12, 77.47, 77.20, 77.07, 76.72, 76.53, 76.40, 76.20, 76.06, 75.69, 75.63, 72.02, 71.85, 71.67, 68.46, 67.91, 65.66, 63.08, 62.79, 62.56, 62.47, 62.36, 62.30, 62.20, 62.08, 61.99, 61.67, 61.50, 61.26, 61.14, 61.04, 60.69, 58.84, 51.22, 51.19, 46.28, 46.14, 45.97, 45.60, 45.43, 44.80, 42.17, 41.93, 41.80, 41.72, 41.60, 41.53, 41.31, 40.04, 39.97, 39.67, 39.62, 39.52, 37.27, 37.15,

35.88, 35.68, 35.41, 35.52, 35.21, 35.14, 35.11, 35.00, 34.90, 34.84, 34.79, 33.71, 33.59, 31.76, 31.44, 31.31, 31.03, 30.98, 30.92, 30.78, 30.65, 30.47, 30.09, 29.89, 29.62, 28.85, 28.74, 28.42, 27.87, 27.75, 27.48, 27.01, 26.92, 26.86, 26.76, 26.28, 26.12, 26.06, 25.74, 25.62, 25.47, 25.27, 25.18, 24.41, 23.80 (q, C-25), 23.49, 23.26, 23.15, 22.76, 22.73, 22.67, 22.63, 22.60, 22.51, 22.48, 22.38, 22.14, 20.06, 19.90, 19.85, 19.63, 19.52, 19.46, 19.38, 19.24, 19.18, 19.11, 18.90, 18.73, 18.58, 18.53, 17.70, 17.66, 17.18, 12.44, 11.94

Next, under a nitrogen atmosphere and water-cooling condition and spending 30 minutes, 50 ml anhydrous tetrahydrofuran solution of methyl 3α,7α,12α-tritetrahydropyranoxy-5β-cholan-24-oate (16.2 g; 23.3 mM) is added dropwise to 250 ml of anhydrous tetrahydrofuran in which aluminium lithium hydride (6.0 g; 158 mM) has been suspended by stirring. After gradually warming up the resulting reaction mixture to room temperature, heating is carried out for 5 hours under reflux.

After cooling spontaneously, the reaction solution is poured carefully in 300 ml of 0.7 N hydrochloric acid and the product is extracted with 600 ml of ethyl acetate. The resulting organic layer is washed with 100 ml of 1 N hydrochloric acid, 100 ml of saturated aqueous solution of sodium hydrogencarbonate, 100 ml of purified water and 100 ml of saturated aqueous solution of sodium chloride and then dried using anhydrous magnesium sulfate. After removing solvent by distillation under a reduced pressure, the thus obtained reaction product in the residue was purified by silica gel column chromatography using n-hexane/ethyl acetate (4:1) as the elution solvent. The product of interest, 3α,7α,12α-tritetrahydropyranoxy-5β-cholan-24-ol, was obtained as a colorless oily substance. (yield; 15.47 g, 97.1%)

3α,7α,12α-tritetrahydropyranoxy-5β-cholan-24-ol:
colorless oil

| Anal. Calcd. for $C_{39}H_{66}O_7$: | | |
|---|---|---|
| | C;72.63, | H;10.00 |
| Found: | C;72.41, | H;10.28 |

$[\alpha]_D^{25}$ = + 10.945 (in $CHCl_3$, c = 0.6865)
Mass (m/z): 646 (M), 562, 460, 443, 394

IR $\nu_{max}^{KBr}$ $cm^{-1}$:

3280, 2934, 2960, 1458, 1374, 1082, 1059
$^1$H-NMR (in $CDCl_3$) δ(ppm):
4.92 - 4.55 (4H, m), 4.03 - 3.81 (5H, m), 3.80 - 3.31 (11H, m), 2.39 - 1.17 (37H, m), 1.03, 1.01 (3H, d, J = 6.79Hz), 0.92, 0.91, 0.89 (3H, t, J = 3.39Hz), 0.70, 0.69, 0.66, 0.65 (3H, dd, J = 2.54Hz, J = 2.83Hz)
$^{13}$C-NMR (in $CDCl_3$) δ(ppm):
101.0, 100.9, 100.7, 98.8, 98.7, 98.5, 98.3, 96.9, 96.7, 96.6, 96.3, 96.1, 95.0, 94.9, 94.8, 94.3, 82.1, 81.9, 77.3, 77.2, 76.9, 76.4, 76.3, 76.2, 75.9, 72.4, 72.1, 71.9, 64.7, 63.6, 63.5, 63.5 (t, C-24), 63.0, 62.8, 62.7, 62.6, 62.4, 62.3, 62.2, 62.1, 61.8, 61.7, 61.5, 61.4, 61.3, 60.4, 46.6, 46.4, 46.3, 46.1, 46.0, 45.9, 42.0, 41.9, 41.8, 41.7, 41.5, 40.2, 40.1, 39.8, 39.8, 39.7, 37.4, 37.3, 36.3, 36.1, 35.9, 35.6, 35.5, 35.4, 35.3, 35.1, 35.0, 35.0, 34.0, 33.9, 32.1, 32.0, 31.9, 31.6, 31.5, 31.1, 31.0, 30.9, 30.1, 29.8, 29.7, 29.6, 29.3, 29.2, 29.0, 28.1, 28.0, 27.8, 27.7, 27.2, 27.1, 27.0, 26.9, 26.5, 26.3, 25.9, 25.8, 25.6, 25.5, 25.2, 24.6, 23.5, 22.9, 22.9, 22.8, 22.7, 22.6, 20.2, 20.1, 20.0, 19.8, 19.8, 19.4, 19.3, 19.3, 19.2, 18.9, 18.8, 18.3, 18.2, 17.8, 14.2, 12.7, 12.1

Anhydrous diethyl ether solution (4.0 ml) of 3α,7α,12α-tritetrahydropyranoxy-5β-cholan-24-ol (647 mg; 1.0 mM) is mixed with 1.3 ml anhydrous pyridine solution of p-toluenesulfonyl chloride (210 mg; 1.2 mM) and the mixture is stirred for 12 hours at room temperature. After diluting the reaction solution with 10 ml of diethyl ether and removing hydrochloric acid salt of pyridine by filtration, the resulting filtrate is washed with 10 ml of 1 N hydrochloric acid, 20 ml of 5% aqueous solution of sodium hydrogencarbonate, 10 ml of water and 10 ml of saturated aqueous solution of sodium chloride. After removing solvent by distillation under a reduced pressure, the thus obtained reaction product in the residue was purified by silica gel column chromatography using n-hexane/ethyl acetate (2:1) as the elution solvent. The product of interest, 3 α,7α,12α-tritetrahydropyranoxy-24-tosyloxy-5β-cholane, is obtained as a colorless oily substance. (yield; 718 mg, 88.9%)

3α,7α,12α-tritetrahydropyranoxy-24-tosyloxy-5β-cholane:

| Anal. Calcd. for $C_{46}H_{70}SO_9$: | | |
| --- | --- | --- |
| | C; 69.55, | H; 8.61 |
| Found: | C; 69.14, | H; 8.83 |

IR $\nu_{max}^{KBr}$ $cm^{-1}$:

3324, 2934, 295, 1661, 1330, 1098
$^{1}$H-NMR (in $CDCl_3$) $\delta$(ppm):
7.81, 7.78 (2H, d, J=7.07Hz), 7.77, 7.36 (2H, d, J=118.2Hz), 4.81 - 4.60 (2H, m), 4.03 - 3.83 (3H, m), 3.75 - 3.71 (1H, brs), 3.70 - 3.62 (1H, brs), 3.61 - 3.38 (3H, brs), 2.45 (2H, s), 2.44 - 0.98 (43H, m), 0.96, 0.94 (2H, d, J=6.6Hz), 0.90, 0.89 (3H, d, J=2.97Hz), 0.86, 0.83 (2H, d, J=6.59Hz), 0.66, 0.65 (2H, d, J=1.98Hz), 0.62, 0.61 (2H, d, J=1.98Hz)
$^{13}$C-NMR (in $CDCl_3$) $\delta$(ppm):
144.6 (s), 129.8 (t), 127.9 (t), 101.0, 100.8, 98.8, 98.7, 98.5, 97.0, 96.7, 96.6, 96.3, 95.3, 95.2, 95.1, 82.1, 81.9, 76.3, 75.9, 72.2, 72.0, 71.3, 71.3, 63.1, 62.8, 62.7, 62.5, 62.3, 61.4, 46.5, 46.3, 46.1, 46.0, 45.8, 42.0, 41.8, 41.7, 41.5, 40.2, 39.8, 37.3, 36.0, 35.8, 35.3, 35.1, 35.1, 31.9, 34.7, 31.2, 31.5, 31.2, 31.0, 30.1, 29.8, 28.1, 27.7, 27.6, 27.2, 27.0, 25.9, 25.8, 25.6, 25.5, 24.6, 23.3, 23.0, 22.8, 22.7, 21.6, 20.3, 20.1, 19.8, 19.7, 19.4, 19.0, 18.8, 18.0, 17.6, 12.6, 12.1

Next, under a nitrogen atmosphere and a water-cooling condition, distilled diethylmalnate (1.82 ml; 12 mM) is added to 5.0 ml of anhydrous dimethylformamide in which sodium hydride (288 mg; 12 mM) has been suspended by stirring, and the mixture is stirred for 30 minutes. To this is added dropwise 20 ml anhydrous dimethylformamide solution of 3α,7α,12α-tritetrahydropyranoxy-24-tosyloxy-5β-cholane (8.0 g; 10.0 mM) spending 10 minutes. After completion of the dropwise addition, the reaction mixture is warmed up to room temperature and the stirring is continued for another 12 hours.

The resulting reaction solution is poured in 50 ml of 0.5 N hydrochloric acid. The product is extracted with 100 ml of ethyl acetate, and the resulting organic layer is washed with 50 ml of 5% aqueous solution of sodium hydrogencarbonate, 50 ml of water and 50 ml of saturated aqueous solution of sodium chloride and then dried using anhydrous magnesium sulfate. After removing solvent by distillation under a reduced pressure, the thus obtained reaction product was purified by silica gel column chromatography using n-hexane/ethyl acetate (2:1) as the elution solvent. The product of interest, diethyl 3α,7α,12α-tritetrahydropyranoxy-5β-cholestane-26,27-dioate, was obtained as a colorless viscous substance. (yield; 5.38 g, 88.9%)
Diethyl 3α,7α,12α-tritetrahydropyranoxy-5β-cholestane-26,27-dioate:

| Anal. Calcd. for $C_{45}H_{73}O_{10}$: | | |
| --- | --- | --- |
| | C; 68.12, | H; 10.13 |
| Found: | C; 69.83, | H; 9.51 |

IR $\nu_{max}^{KBr}$ $cm^{-1}$:

3414, 2952, 1735, 1453, 1378
$^{1}$H-NMR (in $CDCl_3$) $\delta$(ppm):
4.81 - 4.60 (2H, m), 4.32, 4.22, 4.14, 4.06 (4H, q, J=7.02Hz), 4.03 - 3.83 (3H, m), 3.75 - 3.71 (1H, brs), 3.70 - 3.62 (1H, brs), 3.61 - 3.38 (3H, brs), 2.43 - 0.97 (42H, m), 1.35, 1.28, 1.19 (6H, t, J=7.02Hz), 0.96, 0.94 (2H, d, J=6.6Hz), 0.90, 0.89 (3H, d, J=2.97Hz), 0.86, 0.83 (2H, d, J=6.59Hz), 0.66, 0.65 (2H, d, J=1.98Hz), 0.62, 0.61 (2H, d, J=1.98Hz)
$^{13}$C-NMR (in $CDCl_3$) $\delta$(ppm):
202.5 (s, C-26, 27), 101.0, 100.8, 98.9, 98.7, 98.4, 96.7, 96.6, 96.6, 96.3, 95.1, 95.1, 95.0, 82.1, 81.9, 76.3, 75.9, 72.1, 72.0, 71.3, 71.2, 63.0, 62.7, 62.8, 62.4, 61.2 (d, C-25), 61.1, 61.1, 47.9, 46.8, 46.1, 45.9, 45.8,

43.1, 41.8, 41.8, 41.3, 36.2, 35.1, 35.1, 31.9, 31.6, 31.6, 31.6, 31.1, 31.0, 30.1, 29.9, 28.1, 27.7, 27.6, 27.1 (q, C-28, 29), 27.0, 26.0, 25.7, 25.6, 25.4, 24.7, 23.3, 22.4, 22.8, 22.7, 21.1, 20.3, 20.1, 20.0, 19.7, 19.4, 18.8, 18.8, 18.0, 17.6, 13.5, 12.7, 12.0

Next, under a nitrogen atmosphere and water-cooling condition and spending 20 minutes, 25 ml anhydrous tetrahydrofuran solution of 3α,7α,12α-tritetrahydropyranoxy-5β-cholestane-26,27-dioate (774 mg; 1.0 mM) is added dropwise to 30 ml of anhydrous tetrahydrofuran in which aluminium lithium hydride (374 mg; 10.0 mM) has been suspended by stirring. After completion of the dropwise addition, the reaction mixture is warmed up to room temperature and further heated for 5 hours under reflux.

After cooling spontaneously, the reaction solution is poured carefully in 50 ml of 0.7 N hydrochloric acid and the product is extracted with 100 ml of ethyl acetate. The resulting organic layer is washed with 40 ml of 5% aqueous solution of sodium hydrogencarbonate, 40 ml of water and 50 ml of saturated aqueous solution of sodium chloride and then dried using anhydrous magnesium sulfate. After removing solvent by distillation under a reduced pressure, the thus obtained reaction product in the residue was purified by silica gel column chromatography using n-hexane/ethyl acetate (2:1) as the elution solvent. The product of interest, 3α,7α,12α-tritetrahydropyranoxy-5β-cholestane-26,27-diol, was obtained. (yield; 555 mg, 88.0%)

3α,7α,12α-tritetrahydropyranoxy-5β-cholestane-26,27-diol:

| Anal. Calcd. for $C_{41}H_{69}O_8$: | | |
|---|---|---|
| | C; 70.11, | H; 10.92 |
| Found: | C; 71.17, | H; 10.08 |

$$IR\ \nu_{max}^{KBr}\ cm^{-1}:$$

3444, 2910, 1501, 1370, 1112

$^1$H-NMR (in $CDCl_3$) δ(ppm):

4.81 - 4.60 (2H, m), 4.03 - 3.83 (4H, m), 3.72 - 3.70 (2H, brs), 3.68 - 3.48 (8H, m), 2.43 - 0.95 (42H, m), 0.96, 0.93 (2H, d, J = 6.5Hz), 0.90, 0.89 (3H, d, J = 2.97Hz), 0.86, 0.83 (2H, d, J = 6.59Hz), 0.66, 0.64 (2H, d, J = 2.02Hz), 0.62, 0.66 (2H, d, J = 2.02Hz)

$^{13}$C-NMR (in $CDCl_3$) δ(ppm):

101.0, 100.8, 98.8, 98.7, 97.0, 96.7, 96.6, 96.3, 95.3, 95.2, 95.1, 82.1, 81.9, 72.3, 75.9, 72.1, 72.0, 71.3, 71.3, 64.7, 64.5 (t, C-26, 27), 63.0, 62.8, 62.3, 62.2, 62.1, 61.4, 48.2 (d, C-25), 46.6, 46.4, 46.1, 46.0, 45.8, 42.0, 42.0, 41.8, 41.5, 40.8, 39.9, 37.3, 36.0, 35.8, 35.4, 35.1, 35.0, 31.9, 31.8, 31.6, 31.6, 31.1, 31.0, 30.1, 29.8, 28.4, 27.8, 27.6, 27.2, 27.0, 26.9, 25.8, 25.4, 25.0, 24.8, 23.3, 23.0, 22.8, 22.8, 22.7, 21.6, 20.2, 20.1, 19.0, 18.9, 18.1, 17.6, 13.7, 12.6, 12.2

Dowex 50W-X8 (5.0 ml; 5.0 mM) which has been activated with 6 N hydrochloric acid is suspended in 10 ml methanol solution containing 3α,7α,12α-tritetrahydropyranoxy-5β-cholestane-26,27-diol (690 mg; 1.0 mM) and the suspension is stirred for 24 hours at room temperature. After completion of the reaction, the ion exchange resin is removed by filtration and the solvent is distilled off under a reduced pressure. Thereafter, the thus obtained residue was subjected to silica gel column chromatography using lower layer of chloroform/methanol/water system (60:20:8) as elution solvent, to thereby obtain 3α,7α,12α,26,27-pentahydroxy-5β-cholestane.

This compound can be recrystallized from methanol. (yield; 440 mg, 98.2%)

3α,7α,12α,26,27-pentahydroxy-5β-cholestane:

m.p. 197.8 °C, white plate

| Anal. Calcd. for $C_{27}H_{48}O_5 \cdot H_2O$: | | |
|---|---|---|
| | C; 68.90, | H; 10.71 |
| Found: | C; 68.72, | H; 10.42 |

Mass (m/z): 453 (M + $H^+$), 417 (M + $H^+$ -2$H_2O$)

IR $\nu_{max}^{KBr}$ $cm^{-1}$:

3442, 2918, 1463, 1374, 1085, 1024
$^{1}H$-NMR (in $CD_3OD$) $\delta$(ppm):
3.96 (1H, brs), 3.79 (1H, brs), 3.57 (2H, s), 3.55 (2H, s), 3.31 - 3.25 (2H, m), 2.45 - 2.20 (4H, m), 2.10 - 1.08 (27H, m), 1.02, 1.00 (3H, d, J=6.27Hz), 0.91 (3H, s), 0.71 (3H, s)
$^{13}C$-NMR (in $CD_3OD$) $\delta$(ppm):
74.9 (d, C-12), 73.7 (d, C-3), 69.9 (d, C-7), 64.7, 64.5 (t, C-26, 27), 49.1 (d, C-17), 48.2 (s, C-13), 45.2 (d, C-25), 44.0 (d, C-14), 43.7 (d, C-5), 41.8 (d, C-8), 41.2 (d, C-4), 38.3 (t, C-24), 38.0 (d, C-20), 37.3 (t, C-1), 36.7 (S, C-10), 36.6 (t, C-6), 31.9 (t, C-23), 30.4 (t, C-22), 30.2 (t, C-2), 29.6 (t, C-11), 28.6 (t, C-16), 25.5 (d, C-9), 25.0 (q, C-15), 24.0 (q, C-19), 18.6 (q, C-21), 13.8 (q, C-18)

EXAMPLE 7

Freeze-dried powder of head fish bile (20.0 g) was dissolved in water, unnecessary substances were removed with chloroform, the resulting water layer was saturated with sodium chloride and then compounds of interest in the thus saturated solution were extracted with n-butanol. The thus obtained n-butanol extract was treated with Dowex 50W-X8 and then applied to dry type silica gel column chromatography and eluted using lower layer of chloroform/methanol/water system (60:30:12). Each fraction thus eluted was checked using thin layer chromatography, and the fractions were divided into Fraction 1 (6.61 g) and Fraction 2 (7.66 g) based on the RF value of each component.

Thereafter, Fraction 2 (7.66 g) was subjected to dry type silica gel column chromatography using Wako Gel C-100 as fillers and ethyl acetate/acetic acid/water system (90:7:3) as elution solvent to obtain 3.8 g of 3α,7α,12α,26,27-pentahydroxy-5β-cholestane and 3.8 g of 3α,7α,12α,26,27-pentahydroxy-5α-cholestane.
3α,7α,12α,26,27-pentahydroxy-5α-cholestane:
m.p. 198.0°C
$[\alpha]_D^{22}$ = +22.683 (in MeOH, c=0.616)
FAB Mass (m/z);
453 ($M + H^+$), 435 ($M + H^+ - H_2O$), 417 ($M + H^+ - 2H_2O$), 399 ($M + H^+ - 3H_2O$)

IR $\nu_{max}^{KBr}$ $cm^{-1}$:

3360, 2930, 2860, 1712, 1467, 1370, 1070
$^{1}H$-NMR (in $CD_3OD$) $\delta$(ppm):
3.78 - 3.90 (2H, m), 3.55 (4H, d), 3.30 - 3.50 (1H, m), 2.61 - 2.50 (1H, m), 2.34 - 2.18 (2H, m), 2.02 - 1.80 (4H, m), 1.86 - 0.96 (20H, m), 1.03 (3H, s), 0.98 (3H, d, J=6.5Hz), 0.68 (3H, s)
$^{13}C$-NMR (in $CD_3OD$) $\delta$(ppm):
73.9, 70.5, 69.7, 64.7, 64.5, 58.3 (d, C-5), 52.9, 51.7, 49.2, 45.3, 45.1, 44.6, 41.9, 41.8, 40.9, 40.1, 38.2, 38.2, 37.8, 37.0, 33.1, 30.1, 30.1, 25.4, 24.9, 20.0, 13.9

EXAMPLE 8

1) Synthesis of 3α,7α,12α-tritetrahydropyranoxy-5β-cholan-24-al is carried out in accordance with the following procedure i) or ii).

i) PDC method:

The anhydrous $CH_2Cl_2$ 100 ml solution containing 3α,7α,12α-tritetrahydropyranoxy-5β-cholan-24-ol (6.47 g; 10.0 mM) obtained in Example 6 and PDC (5.20 g; 15 mM) is stirred at room temperature for 12 hours. The resulting reaction solution is diluted with 50 ml of $CH_2Cl_2$ and then subjected to suction filtration, followed by concentration of the thus obtained filtrate at 25°C under a reduced pressure. Thereafter, the resulting residue is subjected to $SiO_2$ column chromatography using n-hexane/ethyl acetate (7:3) as the elution solvent system to obtain purified colorless oily product, 3α,7α,12α-tritetrahydropyranoxy-5β-cholan-24-al. (yield; 5.57 g, 86.42%)

ii) PCC-$Al_2O_3$ method:

The anhydrous $CH_2Cl_2$ 100 ml suspension containing 3α,7α,12α-tritetrahydropyranoxy-5β-cholan-24-ol (6.47 g; 10.0 mM) obtained in Example 6 and PCC-$Al_2O_3$ (20.0 g; 20 mM) is stirred at room temperature for 12 hours. The resulting reaction solution is diluted with 50 ml of $CH_2Cl_2$ and then subjected to suction filtration, followed by concentration of the thus obtained filtrate at 25 ° C under a reduced pressure. Thereafter, the resulting residue is subjected to $SiO_2$ column chromatography using n-hexane/ethyl acetate (7:3) as the elution solvent system to obtain purified colorless oily product, 3α,7α,12α-tritetrahydropyranoxy-5β-cholan-24-al. (yield; 5.71 g, 88.6%)

Physicochemical data of 3α,7α,12α-tritetrahydropyranoxy-5β-cholan-24-al:

| Anal. Calcd. for $C_{39}H_{64}O_7 \cdot 5H_2O$: | | |
|---|---|---|
| Found: | C; 63.79,<br>C; 64.02, | H; 10.15<br>H; 10.18 |

Mass (N/z): 446 (M-CO, 2THP), 269, 223, 85, 55

IR $\nu^{KBr}$ $cm^{-1}$: 3450, 2914, 1725, 1450, 1376, 1351, 1197

$^1$H-NMR (in $CDCl_2$) δ(ppm):

9.78, 9.77, 9.76 (1H, t), 5.01 - 4.45 (3H, m), 4.44 - 3.31 (11H, m), 2.59 - 1.13 (43H, m), 1.13 - 0.80 (3H, dd), 0.80 - 0.64 (3H, dd, J = 11.2Hz)

$^{13}$C-NMR (in $CDCl_3$) δ(ppm):

203.1 (d, C-24), 98.7, 96.6, 96.1, 95.1, 95.0, 94.6, 82.1, 77.2, 76.8, 76.3, 75.8, 75.6, 72.0, 71.5, 68.5, 62.9, 62.7, 62.6, 62.3, 62.2, 47.1, 46.5, 46.3, 46.2, 42.0, 41.7, 41.6, 40.8, 40.7, 39.7, 37.3, 35.3, 35.2, 35.1, 35.0, 34.6, 31.6, 31.5, 31.3, 31.2, 31.1, 30.9, 30.8, 30.6, 29.8, 28.4, 29.1, 28.0, 27.9, 27.6, 27.4, 27.1, 26.9, 25.6, 25.5, 25.4, 25.3, 23.6, 23.1, 22.9, 22.7, 20.2, 19.8, 19.7, 19.4, 18.7, 17.9, 17.4, 15.2, 12.6, 12.1

2) The compound obtained by the above procedure i) or ii) (6.45 g; 10.0 mM) and DABCO (2.0 mM) are dissolved in methyl acrylate (1.86 g; 20.0 mM), and the solution sealed in a reaction vessel with a glass stopper is stirred for 3 weeks at room temperature with shading. The resulting reaction solution is diluted with 150 ml of ethyl acetate, washed with 150 ml of purified water and 150 ml of saturated aqueous solution of sodium chloride and then dried using anhydrous magnesium sulfate. After removing solvent by distillation under a reduced pressure, the thus obtained residue was subjected to $SiO_2$ column chromatography using n-hexane/ethyl acetate (5:2) as the elution solvent system to obtain a purified colorless oily compound, methyl 3α,7α,12α-tritetrahydropyranoxy-24-hydroxy-25-en-5β-cholestane-26-oate. (yield; 4.58 g, 61.2%)

Physicochemical data of methyl 3α,7α,12α-tritetrahydropyranoxy-24-hydroxy-25-en-5β-cholestane-26-oate:

$[\alpha]^{24}$: +25.147 (in $CHCl_3$, c = 1.271)

mass (m/z): 731 (M), 645, 628, 562, 545, 527, 461, 443

IR $\nu^{KBr}$ $cm^{-1}$: 3446, 2944, 1720, 1439, 1377, 1074, 1023

$^1$H-NMR (in $CDCl_3$) δ(ppm):

6.22 (1H, S), 5.79, 5.78 (1H, d), 4.82 - 4.53 (3H, S), 3.79 (3H, S), 4.44 - 3.31 (9H, m), 2.60 - 2.11 (2H, m), 2.11 - 1.09 (34H, m), 1.02, 1.00 (3H, dd, J = 6.6Hz), 0.9, 0.89 (6H, dd, J = 2.97Hz), 0.64 - 0.65 (3H, DD, J = 11.2Hz)

$^{13}$C-NMR (in $CDCl_3$) δ(ppm):

167.0 (s, C-26), 142.7, 142.5 (s, C-25), 125.6, 124.7 (t, C-27), 100.9, 100.7, 98.7, 98.6, 98.4, 98.3, 96.5, 96.2, 96.1, 95.0, 94.9, 94.9, 82.1, 84.8, 77.5, 77.2, 76.5, 76.3, 75.8, 75.8, 72.4, 72.2, 72.0, 71.8, 71.8, 62.7, 62.4, 62.3, 62.1, 61.8, 61.6, 61.3, 53.0, 52.1, 51.8, 41.9, 41.9, 41.8, 46.7, 41.6, 41.6, 40.2, 39.8, 39.8, 37.3, 35.9, 35.8, 35.6, 35.5, 35.4, 35.4, 35.3, 35.0, 35.0, 34.0, 33.9, 32.9, 32.7, 32.0, 31.9, 31.5, 31.5, 31.1, 30.9, 30.9, 30.0, 29.7, 28.2, 28.0, 27.7, 27.7, 27.6, 27.2, 27.1, 27.0, 26.9, 26.4, 26.2, 25.9, 25.8, 25.7, 25.6, 24.6 (q, C-28), 23.7, 23.3, 22.8, 22.7, 22.5, 20.0, 19.8, 19.4, 19.0, 18.9, 18.7, 18.3, 18.3, 18.2, 18.1, 12.7, 12.1

3) Synthesis of 3α,7α,12α,24,26-pentahydroxy-25-en-5β-cholestane is carried out in accordance with the following procedure A) or B).

A) Dowex 50W-X8 (20 ml; 20 mM) which has been activated with 6 N hydrochloric acid is added to methanol solution (20 ml) containing the compound obtained in the above step 2) (7.31 g; 10.0 mM) and the mixture is stirred for 20 hours at room temperature. After completion of the reaction, the ion exchange resin is removed by filtration and the solvent is distilled off under a reduced pressure. Thereafter, the thus obtained compound, methyl 3α,7α,12α,24-tetrahydroxy-25-en-5β-cholestane-26-oate, was purified by subjecting the compound to $SiO_2$ column chromatography using lower layer of chloroform/methanol/water system (60:20:8) as elution solvent. This compound can be recrystallized from methanol. (yield; 4.70 g, 98.2%)

Physicochemical data of methyl 3α,7α,12α,24-tetrahydroxy-25-en-5β-cholestane-26-oate:
m.p. 192.2°C, white plate
$[\alpha]^{25}$: +70.038 (in $CH_3OH$, c=0.5125)
Mass (m/z): 460 (M-$H_2O$), 442 (M-2$H_2O$), 423, 406, 393, 271
IR $\nu^{KBr}$ $cm^{-1}$: 3392, 2934, 1705, 1375, 1074, 1039
$^1$H-NMR (in $CD_3OD$) δ(ppm):
6.20 (1H, s), 5.86 (1H, s), 4.41 (1H, M), 3.94 (1H, brs), 3.79 (1H, brs), 3.75 (3H, s), 3.31 - 3.28 (2H, m), 2.32 - 2.10 (4H, m), 2.08 - 1.01 (23H, m) 1.01, 0.98 (3H, d, J=6.59Hz), 0.91 (3H, s), 0.71, 0.70 (3H, d, J=2.64Hz)
$^{13}$C-NMR (in $CD_3OD$) δ(ppm):
169.1 (s, C-26), 146.6, 146.5 (s, C-25), 125.2, 125.0 (t, C-27), 72.4, 71.7 (d, C-24R, 24-S), 74.8 (d, C-12), 73.6 (d, C-3), 69.8 (d, C-7), 50.1 (d, C-5), 48.2 (d, C-13), 43.9 (d, C-14), 43.7 (d, C-5), 41.8 (d, C-8), 41.2 (t, C-4), 37.9 (t, C-20), 37.5 (t, C-1), 37.3 (s, C-10), 36.6 (t, C-6), 35.1, 35.0 (t, C-23), 34.0, 29.5 (t, C-16), 29.5 (s, C-28), 28.6 (t, C-9), 25.0 (t, C-15), 24.0 (t, C-19), 18.9, 18.8 (q, C-21), 13.8 (q, C-18)

Next, under a nitrogen atmosphere and spending 30 minutes, 1.0 M DIBAL-H (25 ml) is added dropwise to the solution obtained by mixing anhydrous tetrahydrofuran solution (15 ml) with the above compound (2.25 g; 5.0 mM) under ice-cooling with stirring. After warming up the resulting reaction mixture to room temperature, the stirring is continued for another 10 hours.

The reaction solution is poured carefully in 100 ml of 1 N hydrochloric acid and the product is extracted with 150 ml of ethyl acetate/n-butanol (1:1). The resulting extract is washed with 50 ml of 5% aqueous solution of sodium hydrogencarbonate and 50 ml of purified water and then solvent is distilled off under a reduced pressure at 50°C. Thereafter, the resulting residue was subjected to $SiO_2$ column chromatography using lower layer of chloroform/methanol/water (60:20:8) as the elution solvent system to obtain a purified colorless oily compound, 3α,7α,12α,24-tetrahydroxy-25-en-5β-cholestane-26-ol.
(yield; 1.42 g, 78.1%)
Physicochemical data of 3α,7α,12α,24-tetrahydroxy-25-en-5β-cholestane-26-ol:

| Anal. Calcd. for $C_{27}H_{45}O_5 \cdot H_2O \cdot 2MeOH$: | | |
|---|---|---|
| | C; 65.63, | H; 10.25 |
| Found: | C; 66.01, | H; 11.58 |

$[\alpha]^{25}$: +16.301 (in $CH_3OH$, c=0.4975)
Mass (m/z): 451 (M + $H^+$), 415 (M + $H^+$ -2$H_2O$)
IR $\nu^{KBr}$ $cm^{-1}$: 3402, 2934, 1704, 1438, 1375, 1704, 1032
$^1$H-NMR (in $CD_3OD$) δ(ppm):
5.12, 5.07 (2H, d, J=12.2Hz), 4.15 - 4.02 (H, brs), 4.02 - 3.83 (1H, brs), 3.83 - 3.69 (1H, brs), 3.69 - 3.39 (1H, m), 3.39 - 3.27 (2H, m), 2.33 - 2.10 (2H, m), 2.10 - 1.21 (23H, m), 1.20 - 0.95 (3H, d, J=6.27Hz), 0.91 (3H, s), 0.71 (3H, s)
$^{13}$C-NMR (in $CD_3OD$) δ(ppm):
153.6, 153.3 (s, C-25), 111.0, 110.8 (t, C-27), 75.7, 75.1 (d, C-24), 74.8 (d, C-12), 73.6 (d, C-3), 69.8 (d, C-7), 63.8, 63.6 (t, C-26), 50.1 (d, C-17), 49.2 (d, C-13), 48.9 (d, C-14), 43.9 (d, C-5), 43.7 (t, C-8), 41.8 (t, C-4), 37.9 (t, C-20), 37.3 (t, C-1), 36.7 (s, C-10), 36.6 (t, C-6), 34.0, 34.0 (t, C-23), 33.9, 33.8 (t, C-22), 31.9 (t, C-2), 30.3 (t, C-11), 29.6 (t, C-16), 28.6 (t, C-9), 25.0 (t, C-15), 24.0 (q, C-19), 18.9 (q, C-21), 13.8 (q, C-18)
B) Under a nitrogen atmosphere and on an ice bath, 1.5 M DIBAL-H (7.7 ml; 11.5 mM) is added dropwise to anhydrous tetrahydrofuran solution (10 ml) containing the compound obtained in the above step 2) (3.51 g; 4.8 mM), with stirring and spending 30 minutes. After warming up the resulting reaction mixture to room temperature, the stirring is continued for another 12 hours.

The reaction solution is poured in 100 ml of 0.5 N hydrochloric acid and the product is extracted with 100 ml of ethyl acetate. The resulting extract is washed with 50 ml of saturated aqueous solution of sodium hydrogencarbonate, 50 ml of purified water and 50 ml of saturated sodium chloride solution and then dried using anhydrous magnesium sulfate. After distilling off the solvent under a reduced pressure, the resulting residue was subjected to $SiO_2$ column chromatography using n-hexane/ethyl acetate (1:1) as the elution solvent system to obtain a purified colorless oily compound, 3α,7α,12α-tritetrahydropyranoxy-24-hydroxy-25-en-5β-cholestane-26-ol. (yield; 1.36 g, 61.7%)

Dowex 50W-X8 (10.0 ml; 10 mM) which has been activated with 6 N hydrochloric acid is added to methanol solution (20 ml) containing the just obtained compound (703 mg; 1.0 mM) and the mixture is stirred for 24 hours at room temperature.

After completion of the reaction, the ion exchange resin is removed by filtration and the solvent is distilled off under a reduced pressure. Thereafter, the thus obtained residue was subjected to $SiO_2$ column chromatography using lower layer of chloroform/methanol/water system (60:20:8) as elution solvent to obtain a purified colorless viscous compound, 3α,7α,12α,24,26-pentahydroxy-25-en-5β-cholestane. (yield; 408 mg, 90.5%)

4) Under a nitrogen atmosphere and shading with water-cooling, 1.0 M borane tetrahydrofuran solution (10.0 ml; 10.0 mM) is added dropwise spending 10 minutes to stirred anhydrous tetrahydrofuran solution (10 ml) containing the compound (450 mg; 1.0 mM) obtained in the above step 3) by the procedure A) or B). After warming up to room temperature, the stirring is continued for additional 6 hours under a shade. After carefully adding 3.0 ml of purified water to the reaction solution, the resulting solution is stirred for 1 hour and then mixed at a stretch with 2.0 ml of 3 M sodium hydroxide solution. After stirring the resulting mixture for 1 hour, 2.4 ml of 30% hydrogen peroxide solution is added dropwise to the stirred mixture spending 15 minutes, followed by further stirring at 40° C for 6 hours. The resulting reaction solution is poured in 100 ml of purified water and extracted with 100 ml of ethyl acetate/n-butanol (1:1). The extract is washed with 100 ml of water and solvents are distilled off under a reduced pressure. The resulting residue is subjected to $SiO_2$ column chromatography using lower layer of chloroform/methanol/water (60:20:8) as the elution solvent system to obtain 336 mg of a purified viscous product. The thus obtained product is dissolved in 3 ml of tetrahydrofuran/pyridine (1:1) solvent system, and the solution is mixed with 3,5-dinitrophenyl isocyanate (1.2 g). After stirring at room temperature for 24 hours, the resulting reaction solution is concentrated under a reduced pressure and then subjected to HPLC [column, SIMIKIRAL OA-4100; solvent system, chloroform/methanol/acetic acid (70:30:1)] to obtain two purified products (330 mg, 345 mg). Both compounds thus obtained are dissolved in anhydrous tetrahydrofuran (3 ml) and subjected to reduction using aluminium lithium hydride (100 mg) under reflux. After spontaneous cooling, the resulting reaction solution is poured in ice water (5 ml) and the reaction products are extracted with 5 ml of ethyl acetate/n-butanol (1:1) and then recrystallized from an ethyl acetate/methanol system to obtain (24R)-3α,7α,12α,24,26,27-hexahydroxy-5β-cholestane (130 mg) and (24S)-3α,7α,12α,24, 26,27-hexahydroxy-5β-cholestane (135 mg).

(24S)-3α,7α,12α,24,26,27-hexahydroxy-5β-cholestane:

Colorless plate; m.p., 187.8° C

$[\alpha]_D^{25}$ = 16.40 (c = 0.5, in methanol)

| Anal. Calcd. for $C_{27}H_{48}O_6 \cdot H_2O$: | | |
|---|---|---|
| | C; 66.63, | H; 10.36 |
| Found: | C; 66.54, | H; 9.90 |

High resolution mass;

432.3220 (error-1.3 mMU) for $C_{27}H_{44}O_4$ ($C_{27}H_{48}O_6$-$2H_2O$)

IR $\nu_{max}^{KBr}$ $cm^{-1}$:

3390, 2930, 1480, 1380, 1074, 1035, 980, 920

$^1$H-NMR (in methanol-$d_4$) δ:

3.96 (1H, broad), 3.79 - 3.58 (6H, m), 3.48 (1H, m), 2.38 - 2.15 (2H, m), 2.10 - 1.20 (23H, m), 1.03 (3H, d, J = 6.01Hz), 0.92 (3H, s), 0.72 (3H, s)

$^{13}$C-NMR (in methanol-$d_4$) δ:

74.9(d), 73.6(d), 73.5(d), 69.8(d), 63.0(t), 62.1(t), 50.1(t), 49.0(d), 48.1(s), 43.8(d), 43.6(d), 41.2(d), 41.1(t), 37.8(d), 37.2(t), 36.6(s), 36.5(t), 34.0(t), 33.0(t), 31.9(t), 30.3(t), 29.5(t), 28.5(d), 25.0(t), 24.0(q), 18.9(q), 13.8(q)

EXAMPLE 9

1) Under a nitrogen atmosphere and spending 30 minutes, an anhydrous tetrahydrofuran solution (25 ml)

of methyl3α,7α,12α-tritetrahydropyranoxy-24-hydroxy-25-en-5β-cholestane-26-oate (730 mg; 1.0 mM) obtained in Example 8-3) is added dropwise to a stirred anhydrous tetrahydrofuran solution (25 ml) of aluminium lithium hydride (374 mg; 10.0 mM), on an ice bath. After completion of the dropwise addition, the resulting mixture is heated under reflux for 5 hours. The reaction solution is poured carefully in 50 ml of 0.7 N hydrochloric acid and the product is extracted with 100 ml of ethyl acetate. The resulting extract is washed with 50 ml of 5% aqueous solution of sodium hydrogencarbonate, 50 ml of purified water and 50 ml of saturated sodium chloride solution and then dried using anhydrous magnesium sulfate. After distilling off the solvent under a reduced pressure, the resulting residue was subjected to $SiO_2$ column chromatography using n-hexane/ethyl acetate (1:1) as the elution solvent system to obtain a purified compound, 3α,7α,12α-tritetrahydropyranoxy-24,26-dihydroxy-5β-cholestan. (yield; 634 mg, 89.1%)

Dowex 50W-X8 (10 ml; 10 mM) which has been activated with 6 N hydrochloric acid is added to methanol solution (20 ml) containing the just obtained compound (634 mg; 1.0 mM) and the mixture is stirred for 24 hours at room temperature. After completion of the reaction, the ion exchange resin is removed by filtration and the solvent is distilled off under a reduced pressure. Thereafter, the thus obtained residue is subjected to $SiO_2$ column chromatography using lower layer of chloroform/methanol/water system (60:20:8) as elution solvent to obtain a purified colorless viscous compound (355 mg).

2) Under a nitrogen atmosphere and spending 30 minutes, an anhydrous tetrahydrofuran solution (25 ml) of methyl 3α,7α,12α-24-tetrahydroxy-25-en-5β-cholestane-26-oate (240 mg; 0.5 mM) obtained in Example 8-4)-A) is added dropwise to a stirred anhydrous tetrahydrofuran solution (25 ml) of aluminium lithium hydride (185 mg; 5.0 mM), on an ice bath. After completion of the dropwise addition, the resulting mixture is gradually warmed up to room temperature and then heated under reflux for 6 hours. After spontaneous cooling, the reaction solution is poured carefully in 150 ml of 0.7 N hydrochloric acid and the product is extracted with ethyl acetate/n-butanol (1:1) solution. The resulting extract is washed with 50 ml of 5% aqueous solution of sodium hydrogencarbonate and 50 ml of purified water. After distilling off the solvent under a reduced pressure, the resulting residue was subjected to $SiO_2$ column chromatography using lower layer of chloroform/methanol/water (60:20:8) as the elution solvent system to obtain a purified colorless viscous compound (192 mg).

3) Under a nitrogen atmosphere and spending 30 minutes, an anhydrous tetrahydrofuran solution (20 ml) of the compound obtained by the procedure A) or B) in Example 8-4) is added dropwise to an ice-cold anhydrous tetrahydrofuran suspension (20 ml) of aluminium lithium hydride (354 mg; 10 mM). After completion of the dropwise addition, the reaction mixture is heated under reflux for 5 hours. After spontaneous cooling, the reaction solution is poured carefully in 100 ml of 0.7 N hydrochloric acid and the product is extracted with 100 ml of ethyl acetate/n-butanol (1:1). The resulting extract is washed with 50 ml of 5% aqueous solution of sodium hydrogencarbonate and 50 ml of purified water. After distilling off the solvent under a reduced pressure, the resulting residue was subjected to $SiO_2$ column chromatography using lower layer of chloroform/methanol/water (60:20:8) as the elution solvent system to obtain a purified product. (yield; 393 g, 90.0%)

The thus obtained products in the above steps 1), 2) and 3) (500 mg) are dissolved in 3 ml of tetrahydrofuran/pyridine (1:1) solvent system, and the solution is mixed with 3,5-dinitrophenyl isocyanate (1.0 g). After stirring at room temperature for 24 hours, the resulting reaction solution is concentrated under a reduced pressure and then subjected to HPLC [column, SIMIKIRAL OA-4100; solvent system, chloroform/methanol/acetic acid (70:30:1)] to obtain two products (430 mg, 443 mg). Both compounds thus obtained are dissolved in anhydrous tetrahydrofuran and subjected to reduction using aluminium lithium hydride (100 mg) under reflux. After spontaneous cooling, the resulting reaction solution is poured in ice water (5 ml) and the reaction products are extracted with 5 ml of ethyl acetate/n-butanol (1:1) and then subjected to $SiO_2$ column chromatography using lower layer of chloroform/methanol/water (60:20:8) as the elution solvent system to obtain (24R)-(25R)-3α,7α,12α,24,26-pentahydroxy-5β-cholestane(224 mg).

(24S)-(25S)-3α,7α,12α,24,26-pentahydroxy-5β-cholestane:

$[\alpha]_D^{25}$ = 35.2 (c = 0.5, in methanol)

| Anal. Calcd. for $C_{27}H_{48}O_5$: | | |
|---|---|---|
| | C; 71.64, | H; 10.69 |
| Found: | C; 71.78, | H; 10.51 |

Ms (m/z): 453 $(M)^+$, 435 $(M\text{-}H_2O)^+$, 417 $(M\text{-}2H_2O)^+$

IR $\nu_{max}^{KBr}$ $cm^{-1}$:

3400, 2920, 1460, 1370, 1080, 1040, 980, 920
$^{1}H$-NMR (in methanol-$d_4$) $\delta$:
3.90 (1H, broad), 3.75 (1H, brs), 3.60 - 3.41 (2H, m), 3.21 (1H, m), 2.51 - 2.04 (2H, m), 2.04 - 0.85 (29H, m), 1.05 (3H, d, J = 6.3Hz), 1.02 (3H, d, J = 6.5Hz), 0.92 (3H, s), 0.75 (3H, s)
$^{13}C$-NMR (in methanol-$d_4$) $\delta$:
75.8(d), 73.8(d), 72.6(d), 68.8(d), 66.8(t), 48.0(d), 47.2(s), 42.9(d), 42.7(d), 41.2(d), 40.8(d), 40.2(t), 37.0(t), 36.7(s), 36.4(t), 35.7(t), 35.6(d), 33.3(t), 32.0(t), 30.0(t), 29.3(t), 28.6(d), 27.6(t), 24.0(q), 23.0(q), 17.9(q), 12.8-(q)

EXAMPLE 10

Under a hydrogen atmosphere, 10% palladium carbon (1.03 g) is activated in ethanol/ethyl acetate (60 ml), and the resulting solution is mixed with benzyl ethyl 3α,7α,12α-triacetoxy-24-keto-5β-cholestane-26,27-dioate (9.54 g; 13.94 mM) obtained in Illustrative Example 2 below. After stirring vigorously at room temperature for 3 days, the resulting reaction mixture is diluted with 50 ml of ethyl acetate and the 10% palladium carbon is removed by filtration. Solvents in the resulting filtrate containing the product of interest were distilled off under a reduced pressure, and the thus obtained residue was subjected to $SiO_2$ column chromatography using n-hexane/ethyl acetate (3:1) as the elution solvent system. The thus obtained product, ethyl 3α,7α,12α-triacetoxy-24-keto-27-nor-5β-cholestane-26-oate, can be recrystallized from ethyl acetate or n-hexane. (yield; 7.583 g, 98.9%)
Physicochemical data of ethyl 3α,7α,12α-triacetoxy-24-keto-27-nor-5β-cholestane-26-oate:
Melting point, 144.6 ° C (white plate)

| Anal. Calcd. for $C_{34}H_{52}O_9$: | | |
|---|---|---|
| | C; 67.53, | H; 8.67 |
| Found: | C; 67.42, | H; 8.67 |

$[\alpha]^{25}$ = +49.742 (in chloroform, c = 504)
Mass (m/z): 431 (M, -$CH_2COOEt$, $2CH_3CO$), 400, 354, 272
IR $\nu^{KBr}$ $cm^{-1}$: 2952, 1735, 1452, 1375, 1255, 1021
$^{1}H$-NMR (in $CDCl_3$) $\delta$(ppm):
5.07 (2H, brs), 4.90 (2H, brs), 4.62 - 4.41 (1H, m), 4.30, 4.22, 4.14, 4.06 (2H, q, J = 7.03Hz), 3.42 (3H, s), 2.49 - 0.72 (21H, m), 2.13 (3H, s), 2.08 (3H, s), 2.04 (3H, s), 1.35, 1.28, 1.12 (3H, t, J = 7.03Hz), 0.92 - 0.80 (7H, m), 0.73 (3H, s)
$^{13}C$-NMR (in $CDCl_3$) $\delta$(ppm):
202.7 (s, C-26), 170.2(s, 2C), 170.1(s), 167.0(s, C-24), 75.7, 74.0, 70.6, 61.2, 59.8, 49.3, 47.6, 45.1, 43.4, 41.0, 39.9, 37.8, 34.7, 34.5, 34.4, 31.3, 29.3, 28.9, 27.2, 26.9, 25.6, 22.8, 22.5, 21.5 (q), 21.4 (q, 2C), 17.7 (q), 14.1, 12.2 (q)

Under a nitrogen atmosphere and spending 10 minutes, anhydrous dimethylformamide solution (5.0 ml) of the just obtained compound (605 mg; 1.0 mM) is added dropwise to anhydrous dimethylformamide solution (10 ml) of sodium hydride (29 mg; 1.2 mM) stirred under ice-cooling, followed by 30 minutes of stirring and subsequent 10 minute-spending dropwise addition of anhydrous dimethylformamide solution (2.0 ml) of methyl iodide (0.15 ml; 2.4 mM). After warming up the resulting reaction mixture to room temperature, the stirring is continued for another 12 hours. The reaction solution is poured in 100 ml of 0.5 N hydrochloric acid and the product is extracted with 100 ml of ethyl acetate. The resulting extract is washed with 20 ml of 5% aqueous solution of sodium hydrogencarbonate, 50 ml of purified water and 50 ml of saturated sodium chloride solution and then dried using anhydrous magnesium sulfate. After distilling off the solvent under a reduced pressure, the resulting residue is subjected to $SiO_2$ column chromatography using n-hexane/ethyl acetate (3:1) as the elution solvent system to obtain a purified product, ethyl 3α,7α,12α-triacetoxy-24-keto-5β-cholestane-26-oate. (yield; 556 mg, 91.2%)
Physicochemical data of ethyl 3α,7α,12α-triacetoxy-24-keto-5β-cholestane-26-oate:
Pearl yellow oil
Mass (m/z): 428 (M-3Ac, -OEt, = 0), 355, 323, 308
IR $\nu^{KBr}$ $cm^{-1}$: 2960, 1731, 1366, 1255, 1021, 893

$^1$H-NMR (in $CDCl_3$) δ(ppm):
5.08 (2H, brs), 4.89 (2H, brs), 4.81 - 4.51 (1H, m), 4.30, 4.22, 4.14, 4.06 (2H, q, J=7.03Hz), 3.62 - 3.38 (1H, q), 2.52 - 0.71 (21H, m), 2.13 (3H, s), 2.08 (3H, s), 2.05 (3H, s), 1.36, 1.28 (3H, d, J=7.00Hz), 1.36, 1.28, 1.27, (3H, t, J=7.03Hz), 0.92 (3H, s), 0.91 - 0.72 (7H, m)
$^{13}$H-NMR (in $CDCl_3$) δ(ppm):
202.5 (s, C-26), 170.4 (s), 170.1 (s), 169.9 (s), 75.3 (d), 74.0 (d), 70.6 (d), 61.1 (t), 52.9 (t, C-25), 52.8 (d), 47.7 (d), 45.1 (d), 43.4 (t), 41.0 (t), 38.3 (t), 38.1 (t), 37.9 (s), 34.7 (t), 34.6 (t), 34.4 (t), 31.3 (t), 29.4 (t), 28.9 (t), 27.8 (q, C-26), 27.1 (t), 26.9 (t), 25.5 (t), 22.5 (q), 21.5 (q), 21.3 (q), 17.7 (q), 14.1 (q), 13.7 (q), 12.7 (q), 12.2 (q)

Under a nitrogen atmosphere, anhydrous tetrahydrofuran solution (10 ml) of the just obtained compound (619 mg; 1.0 mM) is added dropwise to water-cooled stirred anhydrous tetrahydrofuran solution (25 ml) of aluminium lithium hydride (374 mg; 10.0 mM) spending 10 minutes. After completion of the dropwise addition, the resulting mixture is warmed up to room temperature and then heated under reflux for 5 hours. The resulting reaction solution is poured carefully in 100 ml of 0.4 N hydrochloric acid and the product is extracted with 100 ml of ethyl acetate/n-butanol (1:1). The resulting extract is washed with 50 ml of 5% aqueous solution of sodium hydrogencarbonate and 50 ml of purified water. After distilling off the solvent under a reduced pressure at 50°C, the resulting residue was subjected to $SiO_2$ column chromatography using lower layer of chloroform/methanol/water (60:20:8) as the elution solvent system to obtain a colorless viscous product (418 mg).

The thus obtained product is dissolved in 3 ml of tetrahydrofuran/pyridine (1:1) solvent system, and the solution is mixed with 3,5-dinitrophenyl isocyanate (1.0 g). After stirring at room temperature for 24 hours, the resulting reaction solution is concentrated under a reduced pressure and then subjected to HPLC [column, SIMIKIRAL OA-4100; solvent system, chloroform/methanol/ acetic acid (70:30:1)] to obtain two products (231 mg, 242 mg). Both compounds thus obtained are dissolved in anhydrous tetrahydrofuran (3 ml) and subjected to reduction using aluminium lithium hydride (100 mg) under reflux. After spontaneous cooling, the resulting reaction solution is poured in ice water (5 ml) and the reaction products are extracted with 5 ml of ethyl acetate/n-butanol (1:1) and then subjected to $SiO_2$ column chromatography using lower layer of chloroform/methanol/water (60:20:8) as the elution solvent system to obtain purified (24S)-(25R)-3α,7α,12α, 24,26-pentahydroxy-5β-cholestane (224 mg).

(24S)-(25R) 3α,7α,12α,24,26-pentahydroxy-5β-cholestane:
$[\alpha]_D^{25}$ = 45.5 (c=0.5, in methanol)

| Anal. Calcd. for $C_{27}H_{48}O_5$: | | |
|---|---|---|
| | C; 71.64, | H; 10.69 |
| Found: | C; 71.53, | H; 10.47 |

Ms (m/z): 453 $(M)^+$, 435 $(M-H_2O)^+$, 417 $(M-2H_2O)^+$

IR $\nu_{max}^{KBr}$ $cm^{-1}$:

3390, 2925, 1456, 1370, 1070, 1050, 980, 920
$^1$H-NMR (in methanol-$d_4$) δ:
3.95 (1H, broad), 3.77 (1H, brs), 3.62 - 3.43 (2H, m), 3.23 (1H, m), 2.51 - 2.05 (2H, m), 2.05 - 0.75 (29H, m), 1.05 (3H, d, J=6.3Hz), 1.02 (3H, d, J=6.5Hz), 0.90 (3H, s), 0.71 (3H, s)
$^{13}$C-NMR (in methanol-$d_4$) δ:
75.7(d), 73.7(d), 72.5(d), 68.8(d), 66.2(t), 47.2(d), 46.0(s), 42.9(d), 42.5(d), 41.4(d), 41.0(d), 40.7(t), 40.2(d), 39.1(t), 36.8(s), 36.3(t), 35.7(t), 33.2(t), 31.9(t), 30.9(t), 29.3(t), 28.5(d), 27.6(t), 24.6(q), 23.1(q), 18.0(q), 13.0-(q)

## EXAMPLE 11

To anhydrous tetrahydrofuran (1 ml) is dissolved 30 mg of (24R)-(25R)-(+)-24,26-epoxy-5β-cholestane-3α,7α,12α,27-tetrol obtained in Example 4, which is then subjected to reduction using aluminium lithium hydride (20 mg) under reflux. After spontaneous cooling, the resulting reaction solution is poured in ice water (2 ml) and the reaction product is extracted with 3 ml of ethyl acetate/n-butanol (1:1) and then

subjected to $SiO_2$ column chromatography using lower layer of chloroform/methanol/water (60:20:8) as the elution solvent system to obtain a viscous compound, (24R)-(25S)-3α,7α,12α,24,26-pentahydroxy-5β-cholestane (28 mg).
(24R)-(252)-3α,7α,12α,24,26-pentahydroxy-5β-cholestane:
$[\alpha]_D^{25}$ = 36.1 (c = 0.5, in methanol)

| Anal. Calcd. for $C_{27}H_{48}O_5$: | C; 71.64, | H; 10.69 |
| --- | --- | --- |
| Found: | C; 71.71, | H; 10.64 |

Ms (m/z): 453 $(M)^+$, 435 $(M\text{-}H_2O)^+$, 417 $(M\text{-}2H_2O)^+$

$$IR\ \nu_{max}^{KBr}\ cm^{-1}:$$

3390, 2930, 1456, 1376, 1076, 1045, 980, 920
$^1$H-NMR (in methanol-$d_4$) δ:
3.95 (1H, broad), 3.77 (1H, brs), 3.62 - 3.43 (2H, m), 3.23 (1H, m), 2.51 - 2.05 (2H, m), 2.05 - 0.70 (29H, m), 1.04 (3H, d, J = 6.3Hz), 1.01 (3H, d, J = 6.5Hz), 0.90 (3H, s), 0.71 (3H, s)
$^{13}$C-NMR (in methanol-$d_4$) δ:
76.2(d), 73.7(d), 72.5(d), 68.8(d), 66.2(t), 47.2(d), 46.0(s), 42.9(d), 42.5(d), 41.4(d), 41.0(d), 40.7(t), 40.2(d), 39.1(t), 36.8(s), 36.3(t), 35.7(t), 33.2(t), 31.9(t), 30.9(t), 29.3(t), 28.5(d), 27.6(t), 24.6(q), 23.1(q), 18.0(q), 13.0-(q)

## EXAMPLE 12

To anhydrous tetrahydrofuran (1 ml) is dissolved 30 mg of (24R)-(25S)-(+)-24,26-epoxy-5β-cholestane-3α,7α,12α,27-tetrol obtained in Example 3, which is then subjected to reduction using aluminium lithium hydride (20 mg) under reflux. After spontaneous cooling, the resulting reaction solution is poured in ice water (2 ml) and the reaction product is extracted with 3 ml of ethyl acetate/n-butanol (1:1) and then subjected to $SiO_2$ column chromatography using lower layer of chloroform/methanol/water (60:20:8) as the elution solvent system to obtain a viscous compound, (24R)-(25R)-3α,7α,12α,24,26-pentahydroxy-5β-cholestane (26 mg).
(24R)-(25R)-3α,7α,12α,24,26-pentahydroxy-5β-cholestane:
$[\alpha]_D^{25}$ = 28.0 (c = 0.5, in methanol)

| Anal. Calcd. for $C_{27}H_{48}O_5$: | C; 71.64, | H; 10.69 |
| --- | --- | --- |
| Found: | C; 71.60, | H; 10.75 |

Ms (m/z): 453 $(M)^+$, 435 $(M\text{-}H_2O)^+$, 417 $(M\text{-}2H_2O)^+$

$$IR\ \nu_{max}^{KBr}\ cm^{-1}:$$

3395, 2935, 1476, 1380, 1074, 1035, 980, 920
$^1$H-NMR (in methanol-$d_4$) δ:
3.94 (1H, broad), 3.74 (1H, brs), 3.61 - 3.44 (2H, m), 3.23 (1H, m), 2.51 - 2.04 (2H, m), 2.03 - 0.90 (29H, m), 1.04 (3H, d, J = 6.3Hz), 1.02 (3H, d, J = 6.5Hz), 0.87 (3H, s), 0.72 (3H, s)
$^{13}$C-NMR (in methanol-$d_4$) δ:
77.0(d), 73.8(d), 72.6(d), 68.8(d), 66.8(t), 48.0(d), 47.2(s), 42.9(d), 42.7(d), 41.2(d), 40.8(d), 40.2(t), 37.0(t), 36.7(S), 36.4(t), 35.7(t), 35.6(d), 33.3(t), 32.0(t), 30.0(t), 29.3(t), 28.6(d), 27.6(t), 24.0(q), 23.0(q), 17.9(q), 12.8-(q)

ILLUSTRATIVE EXAMPLE 1

Freeze-dried powder of salmon shark bile (12.38 g) was dissolved in water, unnecessary substances were removed by chloroform extraction, the resulting water layer was saturated with sodium chloride and then extraction was carried out with n-butanol. The thus obtained n-butanol extract (Ca. 5.3 g; contains salts) was treated with Dowex 50W-X8 and then applied to silica gel column chromatography and eluted using lower layer of chloroform/ methanol/water system (60:20:8) to obtain a mixed fraction containing (24R)-(+)-3α,7α,12α,24,26,27-hexahydroxy-5β-cholestane. Thereafter, the thus obtained fraction was subjected to recrystallization using a methanol/ethyl acetate system to obtain 1.79 g of (24R)-(+)-3α,7α,12α, 24,26,27-hexahydroxy-5β-cholestane.

(24R)-(+)-3α,7α,12α,24,26,27-hexahydroxy-5β-cholestane:
m.p.: 195.5 - 196.0 °C
$[\alpha]_D^{24}$ = +51.946 (in MeOH, c=0.51)
FAB Mass (m/z):
469 ($M + H^+$), 451 ($M + H^+ -H_2O$), 433 ($M + H^+ -2H_2O$)

$$IR\ \nu_{max}^{KBr}\ cm^{-1}:$$

3322, 2918, 1625, 1450, 1374, 1074, 1009
$^1$H-NMR (in $CD_3OD$) δ(ppm):
3.96 (1H, m), 3.79 - 3.67 (6H, m), 3.34 (1H, m), 2.25 (2H, m), 1.92 - 1.40 (23H, m), 1.03 (3H, d, J=6.3Hz), 0.92 (3H, s), 0.72 (3H, s)
$^{13}$C-NMR (in $CD_3OD$) δ(ppm):
74.9 (d, C-12), 73.6 (d, C-3), 73.1 (d, C-24), 69.9 (d, C-12), 62.8, 62.1 (t, C-26, 27), 49.2 (d, C-5), 48.8 (s, C-13), 48.2 (d, C-25), 44.0 (d, C-14), 43.7 (d, C-5), 41.8 (d, C-8), 41.2 (t, C-4), 37.9 (d, C-20), 37.3 (t, C-1), 36.7 (s, C-10), 36.6 (t, C-6), 34.1 (t, C-23), 33.1 (t, C-22), 31.9 (t, C-2), 30.4 (t, C-11), 29.6 (t, C-16), 28.6 (d, C-9), 25.0 (t, C-5), 24.0 (q, C-19), 18.9 (q, C-21), 13.8 (q, C-18)

ILLUSTRATIVE EXAMPLE 2

A 10 ml portion of pyridine solution of cholic acid (1.0 g; 2.45 mM) is mixed with acetic anhydride (12.0 ml; 109 mM) and stirred at 50 °C for 6 hours. The resulting reaction solution is poured in 50 ml of purified water and extracted with 100 ml of ethanol. The thus obtained extract is washed with 100 ml of 5% aqueous solution of sodium hydrogencarbonate, 100 ml of purified water and 60 ml of saturated sodium chloride solution and then dried using anhydrous magnesium sulfate. After distilling off the solvent under a reduced pressure, the thus obtained residue is subjected to silica gel column chromatography using n-hexane/ethyl acetate (3:1) as the elution solvent system to obtain 3α,7α,12α-triacetoxy-5β-cholan-24-oic acid as white powder. (yield; 1.29 g, 98.2%)

3α,7α,12α-triacetoxy-5β-cholan-24-oic acid:

| Anal. Calcd. for $C_{30}H_{46}O_8$: | | |
|---|---|---|
| | C; 67.39, | H; 8.67 |
| Found: | C; 66.76, | H; 9.02 |

$[\alpha]_D^{25}$ = +71.190 (in $CHCl_3$, c=1.001)
Mass (m/z): 535 ($M + H^+$)

$$IR\ \nu_{max}^{KBr}\ cm^{-1}:$$

2948, 1734, 1447, 1375, 1243, 1025
$^1$H-NMR (in $CDCl_3$) δ(ppm):
5.08 - 4.93 (2H brs), 4.92 - 4.69 (2H, brs), 4.69 - 4.30 (1H, brs), 2.32 - 0.73 (21H, m), 2.13 (3H, s), 2.09 (3H, s), 2.04 (3H, s), 0.92 - 0.88 (7H, m), 0.73 (3H, s)

$^{13}C$-NMR (in $CDCl_3$) δ(ppm):
173.1(s, C-24), 170.2(s), 170.1(s), 170.0(s), 75.3, 74.0, 70.7, 63.9, 56.2, 47.4, 45.1, 43.4, 41.0, 37.9, 34.7, 34.6, 34.4, 31.3, 30.6, 29.6, 28.9, 27.1, 26.9, 25.6, 22.8, 22.5(q), 21.5(q), 21.2(q), 17.5(q), 12.2(q)

Next, spending 10 minutes with stirring, thionyl chloride (3.0 g; 41.1 mM) is added dropwise to 50 ml anhydrous chloroform solution of 3α,7α,12α-triacetoxy-5β-cholan-24-oic acid (4.28 g; 6.0 mM). After completion of the dropwise addition, the resulting mixture is stirred for 12 hours, followed by the removal of solvent by distillation under a reduced pressure. After dissolving the thus obtained residue in 25 ml of anhydrous chloroform, removal of the solvent by distillation under a reduced pressure was repeated three times to obtain the product of interest, 3α,7α,12α-triacetoxy-5β-cholan-24-oyl chloride (3.50 g, 105%). This compound was used in the following reaction step without purification.

Under a nitrogen atmosphere, benzylethyl malonate (1.78 g; 8.0 mM) was added to 10 ml of an ice-cooled anhydrous dimethylformamide to which sodium hydride (197 mg; 8.2 mM) had been added with stirring, and the thus prepared mixture was stirred for 30 minutes. To the resulting reaction mixture is added dropwise 20 ml anhydrous dimethylformamide solution of 3α,7α,12α-triacetoxy-5β-cholan-24-oyl chloride (3.50 g; 6.3 mM) spending 20 minutes. After completion of the dropwise addition, the thus prepared mixture is warmed up to room temperature and stirred for 12 hours. The resulting reaction mixture is poured in 50 ml of 0.5 N hydrochloric acid solution and the formed product is extracted immediately with 150 ml of ethyl acetate. The thus obtained extract is washed with 50 ml of saturated aqueous solution of sodium hydrogencarbonate, 50 ml of purified water and 10 ml of saturated sodium chloride solution and then dried using anhydrous sodium sulfate. After distilling off the solvent at 30 °C under a reduced pressure, the resulting residue was immediately subjected to silica gel column chromatography using n-hexane/ethyl acetate (3:1) as the elution solvent system to obtain benzyl ethyl 3α,7α,12α-triacetoxy-24-keto-5β-cholestane-26,27-dioate as colorless powder. (yield; 3.42 g, 79.2%)

benzyl ethyl 3α,7α,12α-triacetoxy-24-keto-5β-cholestane-26,27-dioate:
m.p.: 87.9 - 88.0 °C

| Anal. Calcd. for $C_{42}H_{58}O_{11}$: | | |
|---|---|---|
| | C; 68.27, | H; 7.91 |
| Found: | C; 67.95, | H; 8.47 |

$[\alpha]_D^{25}$ = +36.868 (in $CHCl_3$, c=0.608)
Mass (m/z): 445 (M-COOBz, COOEt, $2CH_3CO$), 354, 269, 235

IR $\nu_{max}^{KBr}$ $cm^{-1}$:

2940, 2870, 1732, 1375, 1247, 1025, 754
$^1H$-NMR (in $CDCl_3$) δ(ppm):
7.33 - 7.27 (5H, d, J=5.72Hz), 5.17 (2H, s), 5.07 (2H, brs), 4.67 (2H, brs), 4.24 - 3.97 (2H, q, J=7.03Hz), 3.39 (1H, s), 2.13 (3H, s), 2.08 (3H, s), 2.04 (3H, s), 2.34 - 0.73 (21H, m), 1,32, 1.25, 1.17 (3H, t, J=7.03Hz), 0.92 - 0.72 (7H, m), 0.73 (3H, s)
$^{13}C$-NMR (in $CDCl_3$) δ(ppm):
173.7(s, C-26, Ac), 170.1(s, C-27, Ac), 169.1(s, Ac), 128.5(d, C2), 128.3(d, C2), 128.2(s), 125.9(s), 77.2(d), 75.3(d), 74.0, 70.7, 67.1, 61.4, 60.1, 47.5, 45.1, 43.5, 41.6, 41.1, 37.9, 34.7, 34.4, 31.3, 31.2, 30.8, 28.9, 27.2, 26.9, 25.6, 22.9, 22.6, 21.5, 21.4, 17.6(q), 14.3, 14.0, 12.2 (q)

Under a hydrogen atmosphere, 10% palladium carbon (1.03 g) is activated in ethanol/ethyl acetate (60 ml), and the resulting solution is mixed with benzyl ethyl 3α,7α,12α-triacetoxy-24-keto-5β-cholestane-26,27-dioate (9.54 g; 13.94 mM). After stirring vigorously at room temperature for 3 days, the resulting reaction mixture is diluted with 50 ml of ethyl acetate and the 10% palladium carbon is removed by filtration. Solvents in the resulting filtrate containing the product of interest were distilled off under a reduced pressure, and the thus obtained residue was subjected to silica gel column chromatography using n-hexane/ethyl acetate (3:1) as the elution solvent system to obtain (245)-ethyl 3α,7α,12α-triacetoxy-24-keto-27-nor-5β-cholestane-26-oate. This compound, (24S)-ethyl 3α,7α,12α-triacetoxy-24-keto-27-nor-5β-cholestane-26-oate, can be recrystallised from ethyl acetate/n-hexane system. (yield; 7.583 g, 98.9%)

(24S)-ethyl 3α,7α,12α-triacetoxy-24-keto-27-nor-5β-cholestane-26-oate:
m.p. 144.6 °C, white plate

| Anal. Calcd. for $C_{34}H_{52}O_9$: | | |
|---|---|---|
| Found: | C; 67.53,<br>C; 67.42, | H; 8.67<br>H; 8.67 |

$[\alpha]_D^{25}$ = +49.742 (in $CHCl_3$, c=504)
Mass (m/z): 431 (M-$CH_2COOEt$, $2CH_3CO$), 400, 354, 272

IR $\nu_{max}^{KBr}$ $cm^{-1}$:

2952, 1735, 1452, 1375, 1255, 1021
$^1$H-NMR (in $CDCl_3$) δ(ppm):
5.07 (2H, brs), 4.90 (2H, brs), 4.62 - 4.41 (1H, m), 4.30, 4.22, 4.14, 4.06 (2H, q, J=7.03Hz), 3.42 (3H, s), 2.49 - 0.72 (21H, m), 2.13 (3H, s), 2.08 (3H, s), 2.04 (3H, s), 1.35, 1.28, 1.12 (3H, t, J=7.03Hz), 0.92 - 0.80 (7H, m), 0.73 (3H, s)
$^{13}$C-NMR ($CDCl_3$) δ(ppm):
202.7(s, C-26), 170.2(s, 2C), 170.1(s), 167.0(s, C-24), 75.7, 74.0, 70.6, 61.2, 59.8, 49.3, 47.6, 45.1, 43.4, 41.0, 39.9, 37.8, 34.7, 34.5, 34.4, 31.3, 29.3, 28.9, 27.2, 26.9, 25.6, 22.8, 22.5, 21.5 (q), 21.4 (q, 2C), 17.7 (q), 14.1, 12.2 (q)

Under a nitrogen atmosphere and spending 30 minutes, 20 ml of anhydrous tetrahydrofuran solution of ethyl 3α,7α,12α-triacetoxy-24-keto-27-nor-5β-cholestane-26-oate (6.05 g; 10.0 mM) is added dropwise to 50 ml of anhydrous tetrahydrofuran solution of aluminium lithium hydride (1.87 g; 50 mM) stirred under water-cooling. After completion of the dropwise addition, the resulting reaction mixture is warmed up to room temperature and then heated for 5 hours under reflux.

The reaction solution is poured in 300 ml of 0.7 N hydrochloric acid and the product is extracted with 150 ml of ethyl acetate/n-butanol (1:1). The resulting organic layer is washed with 5% aqueous solution of sodium hydrogencarbonate and 50 ml of purified water, and then the solvent is distilled off under a reduced pressure at 50 °C. The thus obtained product, (24R)-Ranol, is purified by silica gel column chromatography using lower layer of chloroform/methanol/water (60:20:8) as the elution solvent system and then recrystallized from ethyl acetate/ methanol system. (yield; 1.91 g, 42.2%)
27-nor-(24R)-(+)-3α,7α,12α,24,26-pentahydroxy-5β-cholestane:
m.p. 195.5 - 196.5 °C

| Anal. Calcd. for $C_{26}H_{46}O_5 \cdot H_2O$: | | |
|---|---|---|
| Found: | C; 68.39,<br>C; 68.50, | H; 10.15<br>H; 10.42 |

$[\alpha]_D^{25}$ = +35 (in $CH_3OH$, c=1.00)
Mass (m/z): 403 (M-$2H_2O$), 385, 289, 271, 226
High resolution mass:
402.3143 error [+ 1.1 mMU] for $C_{26}H_{46}O_5 \cdot 2H_2O$

IR $\nu_{max}^{KBr}$ $cm^{-1}$:

3384, 2930, 1461, 1372, 1074, 1041
$^1$H-NMR (in $CD_3OD$) δ(ppm):
3.95 (1H, brs), 3.80 (1H, brs), 3.71 - 3.64 (2H, m), 3.42 - 3.31 (2H, m), 2.41 - 2.20 (4H, m), 2.08 - 1.09 (27H, m), 1.02, 1.01 (3H, d, J=4.95Hz), 0.92 (3H, s), 0.71 (3H, s)
$^{13}$C-NMR (in $CD_3OD$) δ(ppm):

74.9 (d, C-12), 73.7 (d, C-3), 71.2 (d, C-24), 69.9 (d, C-7), 61.2 (t, C-26), 49.6 (d, C-17), 49.1 (s, C-13), 44.0 (d, C-14), 43.8 (d, C-5), 41.8 (d, C-8), 41.7 (d, C-25), 41.3 (t, C-4), 37.9 (d, C-20), 37.3 (t, C-1), 36.7 (s, C-10), 36.7 (t,C-6), 36.0 (t, C-23), 33.6 (t, C-22), 32.0 (t, c-2), 30.4 (t, C-11), 29.6 (t, c-16), 28.7 (d, C-9), 25.0 (q, C-15), 24.0 (q, C-19), 18.8 (q, C-21), 13.8 (q, C-18)

ILLUSTRATIVE EXAMPLE 3

Under a nitrogen atmosphere and spending 30 minutes, 20 ml of anhydrous tetrahydrofuran solution of (24S)-ethyl 3α,7α,12α-triacetoxy-24-keto-27-nor-5β-cholestane-26-oate (6.05 g; 10.0 mM) obtained in Illustrative Example 2 is added dropwise to 50 ml of anhydrous tetrahydrofuran suspension of aluminium lithium hydride (1.87 g; 50 mM) stirred under water-cooling. After completion of the dropwise addition, the resulting reaction mixture is warmed up to room temperature and then heated for 5 hours under reflux. The reaction solution was carefully poured in 300 ml of 0.7 N hydrochloric acid and the product is extracted with 150 ml of ethyl acetate/n-butanol (1:1). The resulting organic layer is washed with 5% aqueous solution of sodium hydrogencarbonate and 50 ml of purified water, and then the solvent is distilled off under a reduced pressure at 50°C. The thus obtained product, (24S)-Ranol, is purified by silica gel column chromatography using lower layer of chloroform/methanol/water (60:20:8) as the elution solvent system and then recrystallized from ethyl acetate/methanol system. (yield; 1.90 g, 42.0%)
27-nor-(24S)-(+)-3α,7α,12α,24,26-pentahydroxy-5β-cholestane:
m.p. 178.0 - 179.0°C

| Anal. Calcd. for $C_{26}H_{46}O_5 \cdot H_2O$: | | |
|---|---|---|
| | C; 68.39, | H; 10.15 |
| Found: | C; 68.50, | H; 10.42 |

$[\alpha]_D^{25}$ = +30 (in $CH_3OH$, c=1.02)
Mass (m/z): 403 (M-$2H_2O$), 385, 289, 271, 226
High resolution mass:
402.3143 error [+ 1.1 mMU] for $C_{26}H_{46}O_5$-$2H_2O$

$$\mathrm{IR}\ \nu_{max}^{KBr}\ \mathrm{cm}^{-1}:$$

3384, 2930, 1461, 1372, 1074, 1041
$^1$H-NMR (in $CD_3OD$) δ(ppm):
3.95 (1H, brs), 3.80 (1H, brs), 3.71 - 3.64 (2H, m), 3.42 - 3.31 (2H, m), 2.41 - 2.20 (4H, m), 2.08 - 1.09 (27H, m), 1.02, 1.01 (3H, d, J=4.95Hz), 0.92 (3H, s), 0.71 (3H, s)
$^{13}$C-NMR (in $CD_3OD$) δ(ppm):
74.9 (d, C-12), 73.7 (d, C-3), 71.5 (d, C-24), 69.9 (d, C-7), 61.2 (t, C-26), 49.6 (d, C-17), 49.1 (s, C-13), 44.0 (d, C-14), 43.8 (d, C-5), 41.8 (d, C-8), 41.7 (d, C-25), 41.3 (t, C-4), 37.9 (d, C-20), 37.3 (t, C-1), 36.7 (s, C-10), 36.7 (t, C-6), 36.2 (t, C-23), 33.8 (t, C-22), 32.0 (t, C-2) 30.4 (t, C-11), 29.6 (t, C-16), 28.7 (d, C-9), 25.0 (q, C-15), 24.0 (q, C-19), 18.8 (q, C-21), 13.8 (q, C-18)

ILLUSTRATIVE EXAMPLE 4

Freeze-dried powder of mud puppy bile (1.24 g) was dissolved in water, unnecessary substances were removed by chloroform extraction, the resulting water layer was saturated with sodium chloride and then extraction was carried out with ethanol. The thus obtained ethanol extract (Ca. 0.62 g; contains salts) was treated with Dowex 50W-X8 and then applied to silica gel column chromatography and eluted using chloroform/methanol/water (60:20:8) as the elution solvent system to obtain a purified product.

Data on various physical properties of the thus obtained compound were confirmed to coincide with physical properties of 5β-cholestane-3α,7α,12α,25,26-pentol which had been analyzed by instrumental means and disclosed in a report (Tetrahedron Letters, 1977, 687 - 690).

ILLUSTRATIVE EXAMPLE 5

Under a nitrogen atmosphere and ice-cold conditions, 40 ml of anhydrous tetrahydrofuran solution of methyl 3α,7α,12α-trihydroxy-5β-cholan-24-oate (10 g; 23.7 mM) is added dropwise to tetrahydrofuran suspension of aluminium lithium hydride (1.87 g; 50 mM) spending 30 minutes. After completion of the dropwise addition, the resulting reaction mixture is heated for 5 hours under reflux. The reaction solution is carefully poured in 300 ml of 0.7 N hydrochloric acid and the product was extracted with 150 ml of ethyl acetate/n-butanol (1:1). The resulting extract was purified by silica gel column chromatography using lower layer of chloroform/methanol/water (60:20:8) as the elution solvent system and then recrystallized from methanol to obtain 3α,7α,12α,24-tetrahydroxy-5β-cholestane. (yeld; 7.2 g, 77%)

3α,7α,12α,24-tetrahydroxy-5β-cholestane:

m.p. 241.2°C

| Anal. Calcd. for $C_{24}H_{42}O_4 \bullet 1/2\ H_2O$: | | |
|---|---|---|
| | C; 71.46, | H; 10.67 |
| Found: | C; 71.84, | H; 10.60 |

Mass (m/z): 358, 340, 299, 271, 253, 226

$$IR\ \nu_{max}^{KBr}\ cm^{-1}:$$

3250, 2975, 2850, 1483, 1370, 1082

$^1$H-NMR (in $CD_3OD$) δ(ppm):

0.71 (s, 3H), 0.91 (s, 3H), 1.0 (d, 3H, J=7.2Hz), 1.30 - 1.00 (m, 3H), 2.32 - 1.16 (m, 21H), 3.20 - 3.30 (m, 1H), 3.75 - 3.82 (bs, 1H), 3.50 (t, 2H, J=7.0Hz), 3.90 - 3.98 (bs, 1H)

$^{13}$C-NMR (in $CD_3OD$) δ(ppm):

13.8, 18.8, 24.0, 25.0, 28.6, 29.5, 30.3, 31.1, 31.9, 34.0, 36.6, 37.3, 37.8, 41.2, 41.7, 43.7, 43.9, 48.2, 49.0, 49.2, 64.4, 69.8, 73.6, 74.8

ILLUSTRATIVE EXAMPLE 6

A 1 g portion of cholic acid is suspended in 50 ml of tetrahydrofuran, 191 mg of aluminium lithium hydride is added to the suspension and then the thus prepared mixture is subjected to refluxing for 6 hours. After terminating the reaction, 30 ml of 1 N hydrochloric acid was carefully added to the reaction mixture. A 50 ml portion of n-butanol/ethyl acetate (1:1) is added to the mixture, and the resulting organic layer is washed with 50 ml of water and 50 ml of saturated sodium chloride solution. Thereafter, the washed organic layer is concentrated and the thus dried residue is subjected to recrystallization using a methanol/water system to obtain 3α,7α,12α,24-tetrahydroxy-5β-cholan.

The following describes pharmaceutical preparations in which the compounds of the present invention were used.

PREPARATION EXAMPLE 1

| | |
|---|---|
| (1) corn starch | 44 g |
| (2) crystalline cellulose | 40 g |
| (3) calcium carboxymethyl cellulose | 5 g |
| (4) soft silicic anhydride | 0.5 g |
| (5) magnesium stearate | 0.5 g |
| (6) compound obtained in Example 1 | 10 g |
| total | 100 g |

Ingredients (1) to (6) were mixed uniformly in accordance with the above formulation and the mixture was made into compressed tablets, each weighing 200 mg, using a tablet machine.

Since each tablet contains 20 mg of the compound obtained in Example 1, a total of 3 to 10 tablets may be administered per day per adult by dividing the daily dose into several times.

PREPARATION EXAMPLE 2

| | |
|---|---|
| (1) crystalline cellulose | 84.5 g |
| (2) magnesium stearate | 0.5 g |
| (3) calcium carboxymethyl cellulose | 5 g |
| (4) compound obtained in Example 2 | 10 g |
| total | 100 g |

Ingredients (1), (4) and a part of (2) were mixed uniformly in accordance with the above formulation and, after compression-molding the mixture and subsequently crushing the molded product, ingredient (3) and the rest of the ingredient (2) were mixed with the crushed product and the resulting mixture was made into compressed tablets, each weighing 200 mg, using a tablet machine.

Since each tablet contains 20 mg of the compound obtained in Example 2, a total of 3 to 10 tablets may be administered per day per adult by dividing the daily dose into several times.

PREPARATION EXAMPLE 3

| | |
|---|---|
| (1) crystalline cellulose | 34.5 g |
| (2) 10% hydroxypropyl cellulose in ethanol | 50 g |
| (3) calcium carboxymethyl cellulose | 5 g |
| (4) magnesium stearate | 0.5 g |
| (5) compound obtained in Example 3 | 10 g |
| total | 100 g |

Ingredients (1), (2) and (5) were mixed uniformly in accordance with the above formulation, melt-kneaded in usual way and then made into granules using an extrusion granulator. After drying and crushing the granules, the thus crushed product was mixed with ingredients (3) and (4) and the resulting mixture was made into compressed tablets, each weighing 200 mg, using a tablet machine.

Since each tablet contains 20 mg of the compound obtained in Example 3, a total of 3 to 10 tablets may be administered per day per adult by dividing the daily dose into several times.

PREPARATION EXAMPLE 4

| | |
|---|---|
| (1) corn starch | 84 g |
| (2) magnesium stearate | 0.5 g |
| (3) calcium carboxymethyl cellulose | 5 g |
| (4) soft silicic anhydride | 0.5 g |
| (5) compound obtained in Example 4 | 10 g |
| total | 100 g |

Ingredients (1) to (5) were mixed uniformly in accordance with the above formulation, the mixture was compress-molded using a compression molding machine, the molded product was crushed using a crusher and then the crushed product was sieved to obtain granules.

Since 1 g of the granules contain 100 mg of the compound obtained in Example 4, a total of 0.6 to 2 g may be administered per day per adult by dividing the daily dose into several times.

PREPARATION EXAMPLE 5

| | |
|---|---|
| (1) crystalline cellulose | 55 g |
| (2) 10% hydroxypropyl cellulose in ethanol | 35 g |
| (3) compound obtained in Example 5 | 10 g |
| total | 100 g |

Ingredients (1) to (3) were mixed uniformly in accordance with the above formulation and the mixture was melt-kneaded. Thereafter, the kneaded product was subjected to granulation using an extrusion granulator and the granulation product was dried and sieved to obtain granules.

Since 1 g of the granules contain 100 mg of the compound obtained in Example 5, a total of 0.6 to 2 g may be administered per day per adult by dividing the daily dose into several times.

PREPARATION EXAMPLE 6

| | |
|---|---|
| (1) corn starch | 89.5 g |
| (2) soft silicic anhydride | 0.5 g |
| (3) compound obtained in Example 6 | 10 g |
| total | 100 g |

Ingredients (1) to (3) were mixed uniformly in accordance with the above formulation, and 200 mg of the resulting mixture was contained in a No. 2 capsule.

Since each of the capsules thus prepared contains 20 mg of the compound obtained in Example 6, a total of 3 to 10 capsules may be administered per day per adult by dividing the daily dose into several times.

PREPARATION EXAMPLE 7

| | |
|---|---|
| (1) distilled water for injection use | 89.5 g |
| (2) soybean oil | 5 g |
| (3) soybean phospholipid | 2.5 g |
| (4) glycerol | 2 g |
| (5) compound obtained in Example 7 | 1 g |
| total | 100 g |

In accordance with the above formulation, ingredient (5) was dissolved in ingredients (2) and (3), and the resulting solution was mixed with ingredients (1) and (4) and emulsified to obtain injections.

The following test examples are provided to illustrate usefulness of the compounds of the present invention as pharmaceutical drugs.

TEST EXAMPLE 1

In this experiment, 5 to 6-week-old male mice of ddY line (10 individuals per one group) were used after fasting treatment for 24 hours.

Each compound obtained in the present invention was administered intraperitoneally. After 60 minutes of the administration, each mouse was transferred separately into a transparent vessel (capacity, 1,000 ml) equipped with an air exit, and the vessel was aerated with a mixed gas consisting of 96% nitrogen and 4% oxygen at an aeration rate of 5 ℓ/min. Duration from the start of aeration to respiratory arrest (survival time) was measured, with the results shown in Table 1.

Each experimental mouse showed cyanosis after around 15 to 20 seconds of the commencement of aeration of the gas mixture, followed by running, resting, lateral positioning and repetition of clonic spasm in that order. All tested mice died within 180 seconds after the commencement of the gas mixture aeration. Terminal respiration was observed several times prior to the respiratory arrest. Survival time of the control group was roughly in the range of from 96 to 111 seconds.

Compared to the control group, an extending tendency or a significant extending effect was observed in

terms of the survival time in each compound-administered group.

On the basis of these results, each compound of the present invention is considered to be useful as a cerebral function protector.

TABLE 1

| Compound Obtained in: | Dose (mg/kg, i.p.) | Survival Time ±S.E. (second) |
|---|---|---|
| Control | - | 99.8 ± 3.19 |
| Example 1 | 50 | 115.2 ± 5.21 |
| Example 2 | 50 | 111.1 ± 6.35 |
| Example 3 | 50 | 113.2 ± 5.28 |
| Example 4 | 50 | 115.5 ± 3.88 |
| Example 5 | 50 | 110.0 ± 5.10 |
| Example 6 | 50 | 127.5 ± 9.26 |
| Example 7 | 50 | 121.5 ± 6.00 |
| Example 8 | 50 | 144.8 ± 10.27 |
| Example 9 | 50 | 126.5 ± 5.21 |
| Example 10 | 50 | 125.1 ± 3.89 |
| Example 11 | 50 | 121.1 ± 2.60 |
| Example 12 | 50 | 118.1 ± 3.33 |
| Illustrative Example 1 | 50 | 155.4 ± 13.2 |
| Illustrative Example 2 | 50 | 116.3 ± 4.86 |
| Illustrative Example 3 | 50 | 109.2 ± 4.77 |
| Illustrative Example 4 | 50 | 118.0 ± 5.11 |
| Illustrative Example 5 | 50 | 110.5 ± 6.71 |
| Illustrative Example 6 | 50 | 115.4 ± 2.24 |

## TEST EXAMPLE 2

In this experiment, 5 to 6-week-old male mice of ddY line (10 individuals per one group) were used after fasting treatment for 24 hours. After 60 minutes of the administration of each compound, KCN (3.0 mg/kg) was administered by tail intravenous injection. Duration from the commencement of KCN administration to respiratory arrest (survival time) was measured and lethality was calculated, with the results shown in Tables 2 and 3.

In the control group, all tested mice died within 70 seconds after the commencement of the KCN treatment due to respiratory arrest. Compared to the control group, each compound showed significant repression of KCN-induced lethality. Also, compared to the control group, each compound showed an extending tendency or a significant extending effect in terms of the survival time.

On the basis of these results, each compound of the present invention is considered to be useful as a cerebral function protector.

| TABLE 2 | | |
|---|---|---|
| Compound Obtained in: | Dose (mg/kg, i.p.) | Survival Time ± S.E. (second) |
| Control | - | 53.8 ± 4.31 |
| Example 1 | 50 | 95.6 ± 7.12 |
| Example 2 | 50 | 121.8 ± 12.8 |
| Example 3 | 50 | 107.3 ± 8.6 |
| Example 4 | 50 | 98.1 ± 13.1 |
| Example 5 | 50 | 99.5 ± 7.21 |
| Example 6 | 50 | 98.7 ± 8.81 |
| Example 7 | 50 | 89 ± 9.99 |
| Example 8 | 50 | 123.0 ± 7.19 |
| Example 9 | 50 | 101.2 ± 10.1 |
| Example 10 | 50 | 111.1 ± 9.11 |
| Example 11 | 50 | 103.0 ± 7.56 |
| Example 12 | 50 | 121.3 ± 3.21 |
| Illustrative Example 1 | 50 | 159 ± 10.72 |
| Illustrative Example 2 | 50 | 95.3 ± 11.12 |
| Illustrative Example 3 | 50 | 97.2 ± 8.51 |
| Illustrative Example 4 | 50 | 119.0 ± 7.12 |
| Illustrative Example 5 | 50 | 101.1 ± 7.8 |
| Illustrative Example 6 | 50 | 105.3 ± 6.84 |

| TABLE 3 | | |
|---|---|---|
| Compound Obtained in: | Dose (mg/kg, i.p.) | Lethality (%) |
| Control | - | 100 |
| Example 1 | 50 | 90 |
| Example 2 | 50 | 80 |
| Example 3 | 50 | 80 |
| Example 4 | 50 | 80 |
| Example 5 | 50 | 80 |
| Example 6 | 50 | 80 |
| Example 7 | 50 | 80 |
| Example 8 | 50 | 70 |
| Example 9 | 50 | 80 |
| Example 10 | 50 | 80 |
| Example 11 | 50 | 80 |
| Example 12 | 50 | 90 |
| Illustrative Example 1 | 50 | 70 |
| Illustrative Example 2 | 50 | 80 |
| Illustrative Example 3 | 50 | 80 |
| Illustrative Example 4 | 50 | 70 |
| Illustrative Example 5 | 50 | 80 |
| Illustrative Example 6 | 50 | 80 |

TEST EXAMPLE 3

In this experiment, 6-week-old male rats of Wistar line (5 individuals per one group) were used after fasting treatment for 24 hours. After ventrotomy under anesthesia with urethane (0.5 g/kg, i.p.), a canule (SP-1, Natsume Seisakusho Co., Ltd.) was inserted into common bile duct of each rat in retrograde manner. Beginning after 15 minutes of the cannulation, bile has been collected during the subsequent 1 hour.

Immediately thereafter, each compound of the present invention was administered by duodenal injection, and bile samples were collected at one hour interval during the following 5 hours to measure the amount of bile (μg) in each hour. The results are shown in Table 4 in which each of the data is expressed as a ratio of the amount of bile per hour after the compound administration to the amount of bile collected for 1 hour before the administration.

Compared to the control group, each test group showed increased amount of bile in each hour after the administration of respective compound.

On the basis of these results, each compound of the present invention is considered to be useful as a cholagogue.

TABLE 4

| Compound Obtained in: | Dose (mg/kg, i.p) | Ratio of the Amount of Bile ±S.E. (%) Bile Collection Time after Compound Administration (hr) | | | | |
|---|---|---|---|---|---|---|
| | | 0 - 1 | 1 - 1 | 2 - 3 | 3 - 4 | 4 -5 |
| Control | - | 61.8±5.7 | 48.3±5.9 | 40.5±6.8 | 37.0±2.1 | 29.5±2.2 |
| Example 1 | 50 | 63.0±4.2 | 51.1±3.3 | 47.5±5.5 | 46.2±4.7 | 40.1±4.1 |
| Example 2 | 50 | 62.1±2.1 | 55.2±3.3 | 55.1±5.6 | 53.3±4.4 | 41.1±4.8 |
| Example 3 | 50 | 62.3±5.1 | 56.2±5.1 | 54.2±4.9 | 53.5±5.1 | 42.5±3.9 |
| Example 4 | 50 | 63.3±2.2 | 51.3±3.8 | 46.2±3.3 | 45.1±5.1 | 42.4±4.3 |
| Example 5 | 50 | 63.1±1.2 | 52.2±3.2 | 48.5±3.9 | 47.0±4.0 | 41.6±3.3 |
| Example 6 | 50 | 63.0±3.3 | 56.3±3.6 | 51.1±4.6 | 50.1±5.8 | 42.2±4.7 |
| Example 7 | 50 | 62.1±2.1 | 58.1±2.7 | 48.1±3.5 | 47.7±3.3 | 43.5±3.0 |
| Example 8 | 50 | 62.5±2.25 | 55.5±2.33 | 47.1±3.87 | 46.6±3.12 | 40.5±3.31 |
| Example 9 | 50 | 63.8±0.17 | 50.8±2.56 | 51.4±5.10 | 50.2±5.91 | 41.8±6.25 |
| Example 10 | 50 | 62.1±2.97 | 53.1±3.81 | 50.3±2.80 | 50.0±3.07 | 44.9±5.91 |
| Example 11 | 50 | 62.1±1.55 | 50.2±4.21 | 52.1±3.34 | 51.1±2.63 | 40.0±3.55 |
| Example 12 | 50 | 63.8±2.65 | 49.1±4.82 | 49.9±4.12 | 48.8±3.54 | 46.1±4.12 |
| Illust. Example 1 | 50 | 61.0±1.4 | 53.3±3.9 | 52.3±5.2 | 52.0±5.5 | 41.5±5.0 |
| Illust. Example 2 | 50 | 65.2±4.1 | 52.1±2.2 | 50.1±4.8 | 47.1±2.1 | 41.5±3.0 |
| Illust. Example 3 | 50 | 62.5±2.1 | 51.8±4.4 | 51.8±3.3 | 50.8±3.1 | 42.6±3.5 |
| Illust. Example 4 | 50 | 62.1±3.1 | 50.1±3.3 | 49.5±4.6 | 48.1±4.8 | 45.6±6.7 |
| Illust. Example 5 | 50 | 62.5±5.0 | 52.3±2.1 | 50.0±5.0 | 49.0±5.2 | 41.3±2.6 |
| Illust. Example 6 | 50 | 62.4±4.0 | 50.2±3.2 | 49.3±3.7 | 48.7±2.6 | 43.0±2.8 |

TEST EXAMPLE 4

In this experiment, 4-week-old male mice of ddY line (10 individuals per one group) were used after fasting treatment for 24 hours.

After 60 minutes of the administration of each compound of the present invention, each mouse was subjected to ventrotomy under anesthesia with Nembutal® (Dainabot Co., Ltd.; 50 mg/kg, i.p.) to collect

blood from abdominal aorta, and the collected blood sample was immediately checked for its oxygen partial pressure ($PO_2$) and carbon dioxide partial pressure ($PCO_2$) using a blood-aerotonometer (Model 170, Ciba-Corning). The results are shown in Table 5.

Compared to the control group, an increasing tendency or a significant increasing effect was observed in terms of the oxygen partial pressure in each compound-administered group. On the contrary, no difference was observed between the control and test groups with respect to the carbon dioxide partial pressure.

On the basis of these results, each compound of the present invention is considered to be useful as a cell activator.

TABLE 5

| Compound Obtained in: | Dose (mg/kg, i.p.) | Oxygen Partial Pressure (mmHg) | Carbon Dioxide Partial Pressure (mmHg) |
|---|---|---|---|
| Control | - | 73.9 | 41.0 |
| Example 1 | 20 | 91.6 | 42.1 |
| Example 2 | 20 | 86.9 | 43.3 |
| Example 3 | 20 | 86.1 | 45.5 |
| Example 4 | 20 | 85.3 | 39.1 |
| Example 5 | 20 | 87.1 | 39.9 |
| Example 6 | 20 | 83.5 | 40.3 |
| Example 7 | 20 | 81.1 | 41.1 |
| Example 8 | 20 | 98.7 | 42.1 |
| Example 9 | 20 | 88.1 | 43.3 |
| Example 10 | 20 | 87.5 | 42.6 |
| Example 11 | 20 | 86.1 | 40.5 |
| Example 12 | 20 | 87.1 | 41.1 |
| Illustrative Example 1 | 20 | 98.6 | 41.3 |
| Illustrative Example 2 | 20 | 85.4 | 42.1 |
| Illustrative Example 3 | 20 | 89.9 | 40.8 |
| Illustrative Example 4 | 20 | 91.9 | 40.5 |
| Illustrative Example 5 | 20 | 88.1 | 41.2 |
| Illustrative Example 6 | 20 | 89.6 | 40.1 |

## TEST EXAMPLE 5

In this experiment, 5-week-old male rats of SD line (5 individuals per one group) were used after rearing them for 1 week using normal feed, and each compound of the present invention (100 mg/kg) was administered orally once a day for 4 days. Simultaneously with the commencement of the administration, the normal feed was replaced by a high cholesterol feed. After completion of the administration, they were subjected to fasting treatment for 24 hours. Thereafter, each rat was subjected to ventrotomy under anesthesia with Nembutal® (Dainabot Co., Ltd.; 50 mg/kg, i.p.) to collect blood from abdominal aorta, and the collected blood sample was checked for its blood viscosity using a viscometer (Biolyzer, Tokyo Keiki Co., Ltd.). At the same time, total cholesterol (Wako Pure Chemical Industries, Ltd.; Cholesterol E-test wako), triglyceride (Wako Pure Chemical Industries, Ltd.; Triglyceride G-test wako) and GOT (Wako Pure Chemical Industries, Ltd.; GOT-UV test-wako) in serum samples were measured. The results are shown in Tables 6 and 7.

TABLE 6

| Compound (100 mg/ kg/day, p.o.) | Blood Viscosity ±S.E. (c.p.) Shear Rate ($s^{-1}$) 7.5 | 18.8 | 37.5 | 75.0 |
|---|---|---|---|---|
| Control | 13.1±0.3 | 10.1±0.54 | 7.0±0.2 | 6.1±0.2 |
| Example 1 | 8.8±0.5 | 6.7±0.1 | 6.1±0.2 | 4.8±0.2 |
| Example 2 | 8.1±0.2 | 6.8±0.2 | 5.2±0.2 | 4.4±0.4 |
| Example 3 | 7.8±0.3 | 7.1±0.5 | 5.5±0.2 | 4.8±0.4 |
| Example 4 | 8.8±0.2 | 7.2±0.9 | 5.8±0.2 | 4.9±0.5 |
| Example 5 | 8.7±0.1 | 7.1±0.5 | 6.6±0.3 | 5.1±0.4 |
| Example 6 | 8.6±0.1 | 7.2±0.5 | 6.5±0.1 | 5.0±0.4 |
| Example 7 | 8.5±0.1 | 7.1±0.5 | 6.1±0.1 | 4.9±0.2 |
| Example 8 | 7.6±0.1 | 6.6±0.1 | 5.2±0.2 | 4.3±0.3 |
| Example 9 | 9.1±0.2 | 6.9±0.5 | 6.2±0.2 | 4.4±0.5 |
| Example 10 | 8.6±0.1 | 6.8±0.4 | 6.3±0.1 | 4.1±0.1 |
| Example 11 | 8.4±0.1 | 6.9±0.5 | 6.5±0.1 | 4.4±0.4 |
| Example 12 | 8.9±0.1 | 6.8±0.1 | 6.6±0.1 | 5.1±0.4 |
| Illustrative Example 1 | 7.8±0.8 | 6.1±0.4 | 5.3±0.3 | 4.4±0.2 |
| Illustrative Example 2 | 8.8±0.1 | 7.1±0.4 | 6.3±0.1 | 4.8±0.4 |
| Illustrative Example 3 | 8.9±0.1 | 7.2±0.5 | 6.1±0.1 | 5.1±0.4 |
| Illustrative Example 4 | 8.4±0.1 | 7.2±0.4 | 6.0±0.1 | 5.1±0.5 |
| Illustrative Example 5 | 8.3±0.1 | 7.1±0.5 | 5.9±0.1 | 5.2±0.4 |
| Illustrative Example 6 | 8.1±0.1 | 6.7±0.3 | 5.7±0.2 | 4.9±0.3 |

| TABLE 7 | | | |
|---|---|---|---|
| Compound (100 mg/ kg/day, p.o.) | Total Cholesterol ±S.E. (mg/dl) | Triglyceride ±S.E. (mg/dl) | GOT ±S.E. (IU/ℓ) |
| Control | 585 ± 58 | 170 ± 40 | 283 ± 15 |
| Example 1 | 425 ± 60 | 100 ± 25 | 138 ± 10 |
| Example 2 | 435 ± 70 | 95 ± 29 | 150 ± 25 |
| Example 3 | 445 ± 65 | 90 ± 39 | 140 ± 10 |
| Example 4 | 428 ± 49 | 92 ± 45 | 138 ± 9 |
| Example 5 | 405 ± 99 | 91 ± 33 | 141 ± 13 |
| Example 6 | 391 ± 70 | 99 ± 41 | 143 ± 8 |
| Example 7 | 385 ± 58 | 92 ± 31 | 138 ± 18 |
| Example 8 | 410 ± 40 | 87 ± 15 | 140 ± 13 |
| Example 9 | 441 ± 51 | 89 ± 41 | 138 ± 8 |
| Example 10 | 465 ± 59 | 99 ± 29 | 145 ± 7 |
| Example 11 | 433 ± 71 | 110 ± 33 | 151 ± 15 |
| Example 12 | 475 ± 75 | 115 ± 39 | 141 ± 17 |
| Illustrative Example 1 | 390 ± 61 | 90 ± 16 | 128 ± 10 |
| Illustrative Example 2 | 444 ± 61 | 101 ± 42 | 141 ± 6 |
| Illustrative Example 3 | 428 ± 68 | 105 ± 40 | 158 ± 12 |
| Illustrative Example 4 | 450 ± 59 | 108 ± 38 | 116 ± 31 |
| Illustrative Example 5 | 453 ± 49 | 110 ± 29 | 131 ± 21 |
| Illustrative Example 6 | 451 ± 50 | 107 ± 21 | 136 ± 10 |

As shown above, it was confirmed that the blood viscosity level in every compound-administered group was lower than that in the control group. It was confirmed also that levels of total cholesterol, triglyceride and GOT were reduced in the same manner. On the basis of these results, it was confirmed that each compound of the present invention is useful as microcirculation improver, anti-arteriosclerotic agent, anti-hyperlipemic agent and liver function improver.

TEST EXAMPLE 6

Blood was collected from donryu rats of 150 to 200 g in body weight and centrifuged at 1,500 rpm for 5 minutes to obtain red blood cells. The red blood cells thus collected were diluted with 0.15 M phosphate buffer (pH 7.4) to prepare a 5% red blood cell suspension. A 3.8 ml portion of the thus prepared suspension was mixed with 0.2 ml solution of each compound of the present invention which has been adjusted to each level of the concentrations as shown in the following table, and the resulting mixture was shaken thoroughly and incubated at 53 ° C for 20 minutes. Thereafter, the thus incubated mixture was cooled rapidly with tap water and centrifuged at 3,000 rpm for 15 minutes, and absorbance at 540 nm of the resulting supernatant fluid was measured using a spectrophotometer (Hitachi: Type 200-20). Inhibition rate (%) of red blood cell hemolysis at each concentration of the compound was calculated based on the absorbance, with the results shown in Table 8.

As shown in the table, a function of the compounds of the present invention to stabilize red cell membrane was confirmed, because these compounds inhibited thermal hemolysis of red blood cells. On the basis of these results, it was confirmed that these inventive compounds are useful as anti-inflammatory agents.

TABLE 8

| Compound Obtained in: | Hemolysis Inhibition Ratio (%) against Control Concentration of Inventive Compound | | | | |
|---|---|---|---|---|---|
| | 0.5 mM | 0.2 mM | 0.1 mM | 0.05 mM | 0.01 mM |
| Example 1 | 61 | 38 | 23 | 32 | 41 |
| Example 2 | 81 | 61 | 25 | 31 | 38 |
| Example 3 | 70 | 51 | 24 | 30 | 33 |
| Example 4 | 48 | 44 | 31 | 21 | 27 |
| Example 5 | 61 | 41 | 30 | 19 | 23 |
| Example 6 | 62 | 41 | 20 | 25 | 33 |
| Example 7 | 70 | 61 | 31 | 21 | 32 |
| Example 8 | 60 | 50 | 37 | 20 | 27 |
| Example 9 | 65 | 51 | 38 | 23 | 28 |
| Example 10 | 58 | 44 | 31 | 21 | 24 |
| Example 11 | 51 | 43 | 30 | 18 | 20 |
| Example 12 | 55 | 48 | 33 | 20 | 24 |
| Illustrative Example 1 | 80 | 53 | 42 | 20 | 23 |
| Illustrative Example 2 | 71 | 60 | 38 | 23 | 31 |
| Illustrative Example 3 | 61 | 41 | 22 | 31 | 38 |
| Illustrative Example 4 | 49 | 42 | 41 | 25 | 27 |
| Illustrative Example 5 | 58 | 51 | 33 | 21 | 25 |
| Illustrative Example 6 | 59 | 51 | 38 | 22 | 24 |

TEST EXAMPLE 6

In this experiment, 8-week-old male rats of Wistar line (5 individuals per one group) were used after rearing them for 1 week with normal feed. Streptozotocin (40 mg/kg) was administered by tail intravenous injection to obtain rats of diabetes mellitus. Beginning after 1 week of the streptozotocin administration, oral administration of each test drug has been carried out for 1 week, followed by 24 hours of fasting. Thereafter, blood sample was collected from lower abdominal vena cava under anesthesia with Nembutal®

(1 ml/kg, i.p.). Serum samples were prepared from the thus collected blood samples and checked for their blood sugar levels, with the results shown in Table 9.

TABLE 9

| Compound Obtained in: | Dose (mg/kg, i.o.) | Blood Sugar Level ± S.E. (mg/dl) |
|---|---|---|
| Control | - | 350 ± 20.1 |
| Example 1 | 50 | 120 ± 11.2 |
| Example 2 | 50 | 125 ± 8.8 |
| Example 3 | 50 | 143 ± 6.9 |
| Example 4 | 50 | 181 ± 8.9 |
| Example 5 | 50 | 122 ± 7.8 |
| Example 6 | 50 | 138 ± 6.8 |
| Example 7 | 50 | 161 ± 5.5 |
| Example 8 | 50 | 112 ± 8.4 |
| Example 9 | 50 | 125 ± 6.6 |
| Example 10 | 50 | 133 ± 3.8 |
| Example 11 | 50 | 187 ± 2.5 |
| Example 12 | 50 | 163 ± 3.8 |
| Illustrative Example 1 | 50 | 177 ± 5.5 |
| Illustrative Example 2 | 50 | 171 ± 4.7 |
| Illustrative Example 3 | 50 | 165 ± 6.6 |
| Illustrative Example 4 | 50 | 133 ± 5.4 |
| Illustrative Example 5 | 50 | 170 ± 7.2 |
| Illustrative Example 6 | 50 | 159 ± 5.7 |

TEST EXAMPLE 7

In this experiment, 12-week-old male rats of SHR line (5 to 6 individuals per one group) were used after rearing them for 1 week with normal feed. Oral administration of each test drug has been carried out for 2 weeks and then blood pressure (mmHg) was measured using a small animal hemomanometer (UR-1000). The results are shown in Table 10.

| TABLE 10 | | | |
|---|---|---|---|
| Compound Obtained in: | Dose (mg/kg, p.o.) | Blood Pressure ±S.E. (mmHg) | |
| | | Maximum | Average |
| Control | - | 227 ± 3.5 | 179 ± 2.5 |
| Example 1 | 10 | 203 ± 2.8 | 151 ± 6.6 |
| Example 2 | 10 | 189 ± 6.5 | 149 ± 6.3 |
| Example 3 | 10 | 200 ± 7.7 | 150 ± 3.7 |
| Example 4 | 10 | 205 ± 7.8 | 153 ± 2.8 |
| Example 5 | 10 | 181 ± 3.5 | 148 ± 3.5 |
| Example 6 | 10 | 178 ± 3.3 | 131 ± 3.7 |
| Example 7 | 10 | 198 ± 3.7 | 155 ± 3.6 |
| Example 8 | 10 | 180 ± 6.6 | 151 ± 9.5 |
| Example 9 | 10 | 199 ± 5.4 | 150 ± 8.9 |
| Example 10 | 10 | 191 ± 7.2 | 155 ± 9.5 |
| Example 11 | 10 | 188 ± 3.8 | 141 ± 9.1 |
| Example 12 | 10 | 173 ± 3.6 | 138 ± 9.5 |
| Illustrative Example 1 | 10 | 205 ± 2.1 | 139 ± 8.5 |
| Illustrative Example 2 | 10 | 200 ± 3.5 | 145 ± 8.1 |
| Illustrative Example 3 | 10 | 199 ± 2.6 | 141 ± 3.2 |
| Illustrative Example 4 | 10 | 181 ± 2.5 | 144 ± 4.4 |
| Illustrative Example 5 | 10 | 185 ± 7.7 | 151 ± 4.6 |
| Illustrative Example 6 | 10 | 187 ± 6.3 | 148 ± 8.4 |

TEST EXAMPLE 8

In this experiment, 9-week-old male rats of Wistar line (5 to 6 individuals per one group) were used after rearing them for 1 week with normal feed. After 24 hours of fasting, each test drug was administered orally and, 90 minutes thereafter, blood sample was collected from lower abdominal vena cava of each rat under anesthesia with Nembutal® (1 ml/kg, i.p.). Blood samples obtained in this way were checked for their blood clotting time and erythrocyte deformability.

Measuring procedures are as follows.

Blood clotting time ... Collected blood sample is centrifuged at 3,500 rpm for 10 minutes to obtain blood plasma. The blood plasma is pre-incubated for 1 minute in a water bath controlled at 37° C, mixed with calcium chloride and then shaken at 20 second intervals to measure a time required for its clotting.

Erythrocyte deformability ... The collected blood is extruded from a syringe with a constant flow of He in a holder equipped with a filter (5 µm). Weight of blood and the number of erythrocytes filtered during a fixed time are measured and the deformability is expressed based on the number of filtered erythrocytes. The results of these measurements are shown respectively in Tables 11 and 12.

TABLE 11

| Compound Obtained in: | Blood Plasma Clotting Time | |
|---|---|---|
| | Dose (mg/kg, p.o.) | Plasma Clotting Time ± S.E. (second) |
| Control | - | 141 ± 2.5 |
| Example 1 | 30 | 210 ± 2.1 |
| Example 2 | 30 | 205 ± 2.5 |
| Example 3 | 30 | 195 ± 1.9 |
| Example 4 | 30 | 188 ± 1.8 |
| Example 5 | 30 | 195 ± 2.5 |
| Example 6 | 30 | 215 ± 2.5 |
| Example 7 | 30 | 222 ± 2.2 |
| Example 8 | 30 | 207 ± 2.7 |
| Example 9 | 30 | 244 ± 2.3 |
| Example 10 | 30 | 221 ± 2.5 |
| Example 11 | 30 | 177 ± 2.6 |
| Example 12 | 30 | 187 ± 3.8 |
| Illustrative Example 1 | 30 | 198 ± 3.3 |
| Illustrative Example 2 | 30 | 201 ± 4.1 |
| Illustrative Example 3 | 30 | 213 ± 5.5 |
| Illustrative Example 4 | 30 | 205 ± 6.1 |
| Illustrative Example 5 | 30 | 198 ± 2.1 |
| Illustrative Example 6 | 30 | 201 ± 3.9 |

TABLE 12

| Compound Obtained in: | Erythrocyte Deformation | |
|---|---|---|
| | Dose (mg/kg, p.o.) | Erythrocyte counts ±S.E. ($\times 10^7$) |
| Control | - | 2.67 ± 0.51 |
| Example 1 | 30 | 3.51 ± 0.77 |
| Example 2 | 30 | 3.72 ± 0.38 |
| Example 3 | 30 | 3.55 ± 0.66 |
| Example 4 | 30 | 3.97 ± 0.39 |
| Example 5 | 30 | 4.10 ± 0.55 |
| Example 6 | 30 | 3.81 ± 0.44 |
| Example 7 | 30 | 4.02 ± 0.37 |
| Example 8 | 30 | 4.53 ± 0.82 |
| Example 9 | 30 | 4.11 ± 0.44 |
| Example 10 | 30 | 4.41 ± 0.54 |
| Example 11 | 30 | 3.95 ± 0.64 |
| Example 12 | 30 | 3.80 ± 0.78 |
| Illustrative Example 1 | 30 | 3.72 ± 0.55 |
| Illustrative Example 2 | 30 | 3.62 ± 0.46 |
| Illustrative Example 3 | 30 | 3.81 ± 0.23 |
| Illustrative Example 4 | 30 | 3.93 ± 0.38 |
| Illustrative Example 5 | 30 | 3.51 ± 0.65 |
| Illustrative Example 6 | 30 | 3.71 ± 0.48 |

TEST EXAMPLE 9

Each of male SD rats of 130 to 210 g in body weight was anesthetized with Nembutal® (50 mg/kg, i.p.) and subjected to median incision ventrotomy to release about a 2 cm portion of abdominal vena cava below the renal vein. Blood flow of the thus released vena cava was temporarily stopped for 5 minutes with 8 hemostatic clips. After recirculating blood for 5 minutes by removing the hemostatic clips, aorta was ligated at a position just below the renal vein and the median-incised part was sutured. Two hours after the ligature, the thus treated rat was again subjected to ventrotomy to excise abdominal vena cava. The thus excised blood vessel was cut open on a filter paper which had been moistened with physiological saline, in order to take out thrombus. The thrombus thus obtained was washed with physiological saline to remove adhered blood and the like substances and then dried at 80°C for 12 hours or longer to measure its dry weight. In this instance, each drug to be tested was orally administered 90 minutes before the clipping to examine its effect by single administration on the formation of thrombus. Separately from this, each test drug was administered to 6-week-old male SD rat continuously for 7 days, thrombus was prepared in the eighth day in the same manner as described above and dry weight of the thrombus was measured, in order to examine effect of the continuous 7 day administration on the thrombus formation. The results are shown in Table 13.

TABLE 13

| Compound Obtained in: | Antithrombotic Effect | |
|---|---|---|
| | Dose (mg/kg, p.o.) | Thrombus weight ±S.E. (mg) |
| Control | - | 18.5 ± 2.21 |
| Example 1 | 30 | 12.6 ± 2.53 |
| Example 2 | 30 | 13.3 ± 3.25 |
| Example 3 | 30 | 13.5 ± 1.22 |
| Example 4 | 30 | 11.2 ± 3.81 |
| Example 5 | 30 | 11.2 ± 3.12 |
| Example 6 | 30 | 13.5 ± 2.11 |
| Example 7 | 30 | 12.2 ± 1.33 |
| Example 8 | 30 | 10.5 ± 1.43 |
| Example 9 | 30 | 12.5 ± 2.44 |
| Example 10 | 30 | 13.8 ± 2.85 |
| Example 11 | 30 | 14.4 ± 2.01 |
| Example 12 | 30 | 12.2 ± 1.88 |
| Illustrative Example 1 | 30 | 15.1 ± 1.98 |
| Illustrative Example 2 | 30 | 13.4 ± 2.38 |
| Illustrative Example 3 | 30 | 12.3 ± 3.55 |
| Illustrative Example 4 | 30 | 14.1 ± 2.11 |
| Illustrative Example 5 | 30 | 13.8 ± 3.77 |
| Illustrative Example 6 | 30 | 12.4 ± 2.39 |

TEST EXAMPLE 10

Each drug to be tested was orally administered to each male rat which has been subjected to fasting treatment for 18 to 24 hours. After 90 minutes of the administration, median incision ventrotomy was carried out under ether anesthesia to collect 9 volumes of blood using a syringe filled with 1 volume of 3.8% sodium citrate solution (3.8% sodium citrate solution:blood = 1:9). After thorough mixing, the thus prepared mixture was centrifuged at 800 rpm for 10 minutes at room temperature to collect the resulting supernatant fluid as platelet rich plasma (PRP). The residual portion was centrifuged at 3,000 rpm for 15 minutes at room temperature to collect the resulting supernatant fluid as platelet poor plasma (PPP). The PRP was diluted with the PPP to adjust the number of platelets to $3\times10^5 \pm 2\times10^4$ cells/µl. A 450 µl portion of the thus diluted PRP was incubated at 37°C for 3 minutes and then mixed with 50 µl of $1\times10^{-5}$ M of ADP or $1\times10^{-5}$ g/ml of collagen. Thereafter, platelet aggregation test of the mixture was carried out using an aggregometer by measuring transmittance at 940 nm. The results are shown in Table 14.

TABLE 14

| Compound Obtained in: | Dose (mg/kg, p.o.) | Platelet Aggregation Ratio ±S.E. (%) | |
|---|---|---|---|
| | | ADP ($1 \times 10^{-5}$ M) | Collagen ($1 \times 10^{-5}$ g/ml) |
| Control | - | 61.0 ± 1.01 | 65.5 ± 2.22 |
| Example 1 | 30 | 45.1 ± 1.31 | 41.1 ± 1.34 |
| Example 2 | 30 | 40.1 ± 2.51 | 46.5 ± 1.81 |
| Example 3 | 30 | 42.5 ± 3.81 | 42.5 ± 1.85 |
| Example 4 | 30 | 46.1 ± 2.65 | 47.7 ± 3.88 |
| Example 5 | 30 | 48.5 ± 3.31 | 48.1 ± 2.65 |
| Example 6 | 30 | 50.1 ± 2.33 | 42.3 ± 3.83 |
| Example 7 | 30 | 44.3 ± 2.43 | 42.1 ± 3.15 |
| Example 8 | 30 | 41.8 ± 1.81 | 46.6 ± 3.81 |
| Example 9 | 30 | 41.1 ± 1.21 | 49.1 ± 1.11 |
| Example 10 | 30 | 42.2 ± 2.33 | 48.3 ± 1.65 |
| Example 11 | 30 | 43.3 ± 2.81 | 40.8 ± 1.90 |
| Example 12 | 30 | 46.5 ± 5.82 | 41.3 ± 3.81 |
| Illustrative Example 1 | 30 | 50.3 ± 1.32 | 43.8 ± 3.99 |
| Illustrative Example 2 | 30 | 51.5 ± 1.55 | 44.4 ± 4.51 |
| Illustrative Example 3 | 30 | 50.3 ± 1.38 | 45.5 ± 2.66 |
| Illustrative Example 4 | 30 | 42.5 ± 1.65 | 48.7 ± 2.53 |
| Illustrative Example 5 | 30 | 44.8 ± 3.62 | 49.9 ± 4.85 |
| Illustrative Example 6 | 30 | 43.5 ± 3.26 | 47.7 ± 2.58 |

TEST EXAMPLE 11

In this experiment, 30-week-old male mice of ddY line (10 individuals per one group) were used after pre-rearing for 1 week.

Each of the compounds obtained in Examples 1 to 12 and Illustrative Examples 1 to 6 was suspended in 1% Tween 80 in such an amount that said compound can be administered at a dose of 40 mg/20 b.w. when the suspension was administered at a dose of 0.1 ml/20 g b.w. In both of its oral administration and intraabdominal injection, no death was found over 14 days after its single administration.

INDUSTRIAL APPLICABILITY

Drugs which contain bile alcohols represented by the general formula (I) or (II) of the present invention as the active ingredient are useful as a cerebral function protector, a cholagogue, a cell activator, a microcirculation improver, an anti-arteriosclerotic agent, an anti-hyperlipemic agent, a liver function improver, an anti-inflammatory agent, an anti-thrombotic agent and an anti-hypertensive agent.

While the invention has been described in detail and with reference to specific embodiments thereof, it will be apparent to one skilled in the art that various changes and modifications can be made therein without departing from the spirit and scope thereof.

**Claims**

**1.** A bile alcohol represented by the following general formula (I):

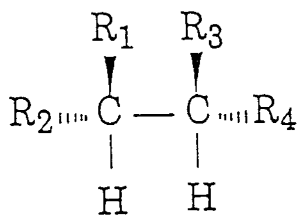

(I)

wherein one of the $R_1$ and $R_2$ is a group represented by the following formula (A) or (B);

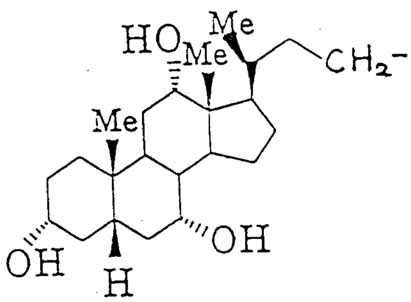

(A)

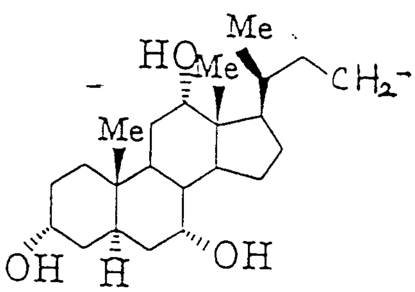

(B)

and the other is -OH or -H, each of $R_3$ and $R_4$ is $-CH_2OH$, $-CH_3$ or $-CH_2OSO_3Na$, in which $R_2$ and $R_3$ may combine with each other to be $-OCH_2-$, $R_2$ and $R_4$ may combine with each other to be $-OCH_2-$ and $R_3$ and $R_4$ may combine with each other to be $-CH_2OCH_2-$, provided that $R_3$ and $R_4$ are not $-CH_3$ at the same time and that $R_1$ is -OH or -H and $R_2$ is a group represented by the formula (A) or (B) when $R_3$ and $R_4$ are both $-CH_2OH$ at the same time.

**2.** The bile alcohol according to claim 1, wherein $R_1$ is a group represented by the formula (A) or (B), $R_2$ is -H or -OH, each of $R_3$ and $R_4$ is $-CH_2OH$, $-CH_3$ or $-CH_2OSO_3Na$, in which $R_2$ and $R_3$ may combine with each other to be $-OCH_2-$, $R_2$ and $R_4$ may combine with each other to be $-OCH_2-$and $R_3$ and $R_4$ may combine with each other to be $-CH_2OCH_2-$.

**3.** The bile alcohol according to claim 2, wherein $R_1$ is a group represented by the formula (A) or (B), $R_2$ is -OH or -H and each of $R_3$ and $R_4$ is $-CH_2OH$, $-CH_3$ or $-CH_2OSO_3Na$.

**4.** A cerebral function protector which comprises a bile alcohol as the active ingredient represented by the following general formula (II):

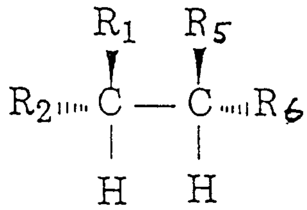

(II)

wherein $R_1$ is a group represented by the following formula (A) or (B);

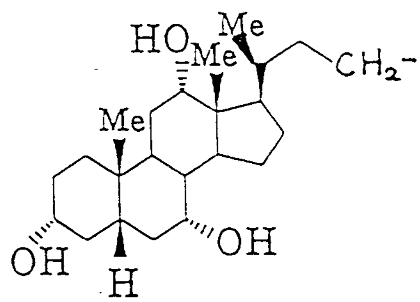

(A)

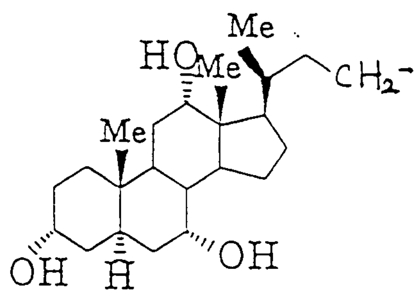

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

**5.** A cholagogue which comprises a bile alcohol as the active ingredient represented by the following general formula (II):

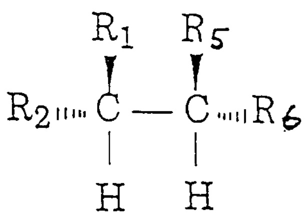

(II)

wherein $R_1$ is a group represented by the following formula (A) or (B);

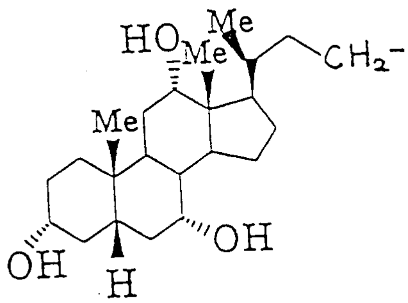

(A)

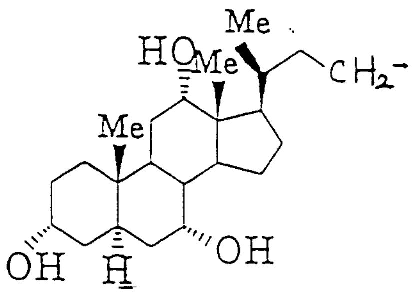

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

**6.** A cell activator which comprises a bile alcohol as the active ingredient represented by the following general formula (II):

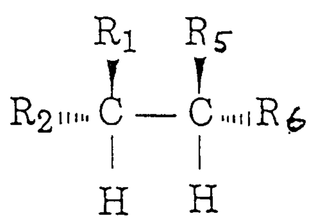

(II)

wherein $R_1$ is a group represented by the following formula (A) or (B);

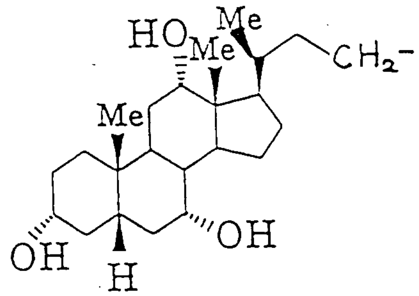

(A)

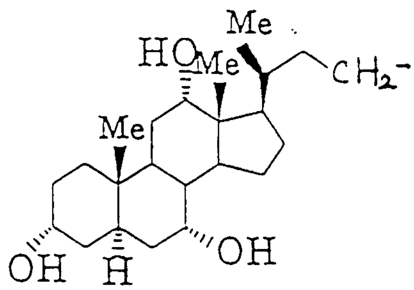

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

**7.** A microcirculation improver which comprises a bile alcohol as the active ingredient represented by the following general formula (II):

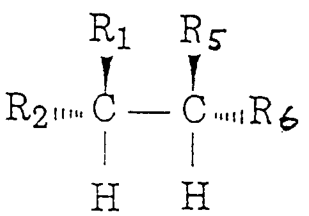

(II)

wherein $R_1$ is a group represented by the following formula (A) or (B);

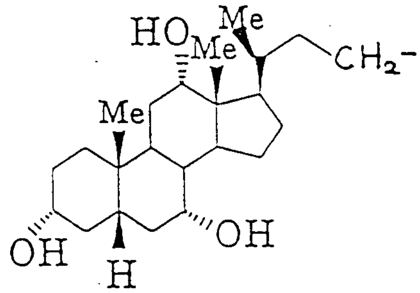

(A)

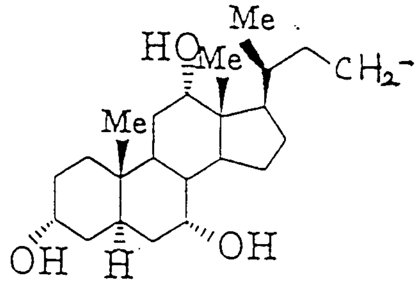

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

**8.** An anti-arteriosclerotic agent which comprises a bile alcohol as the active ingredient represented by the following general formula (II):

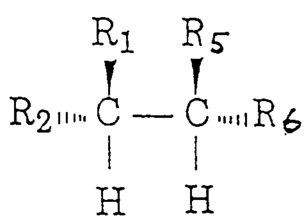

(II)

wherein $R_1$ is a group represented by the following formula (A) or (B);

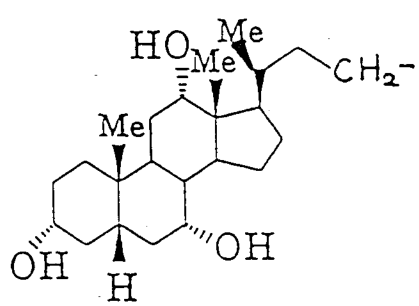

(A)

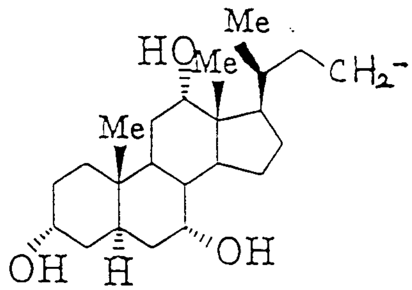

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

9. An anti-hyperlipemic agent which comprises a bile alcohol as the active ingredient represented by the following general formula (II):

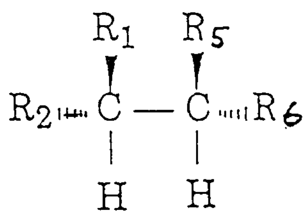

(II)

wherein $R_1$ is a group represented by the following formula (A) or (B);

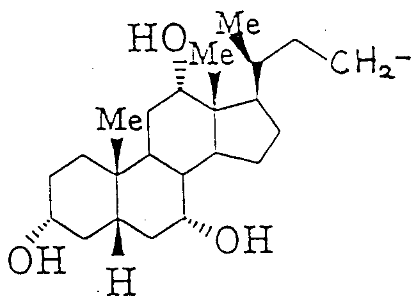

(A)

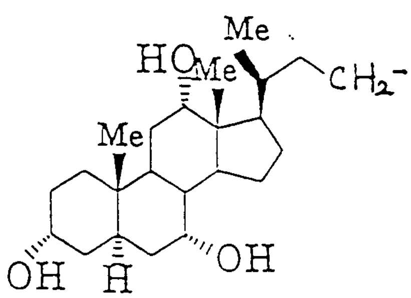

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

10. A liver function improver which comprises a bile alcohol as the active ingredient represented by the following general formula (II):

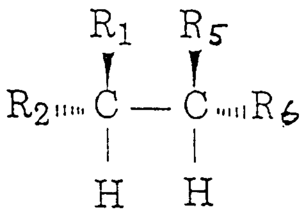

(II)

wherein $R_1$ is a group represented by the following formula (A) or (B);

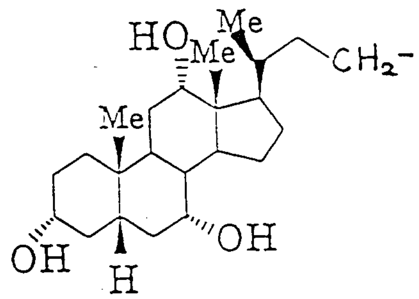

(A)

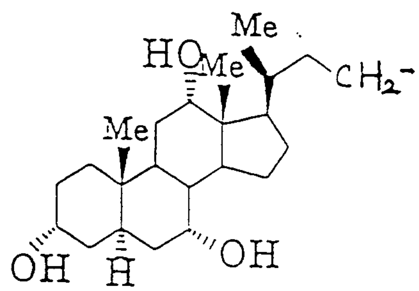

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

11. An anti-inflammatory agent which comprises a bile alcohol as the active ingredient represented by the following general formula (II):

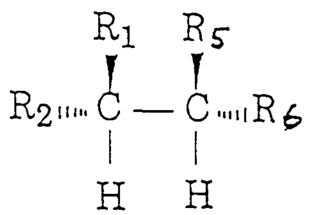

(II)

wherein $R_1$ is a group represented by the following formula (A) or (B);

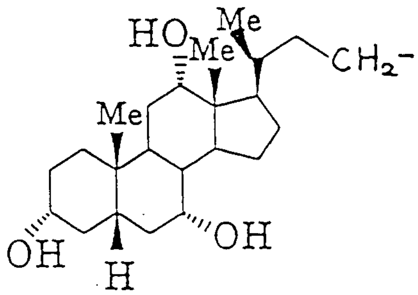

(A)

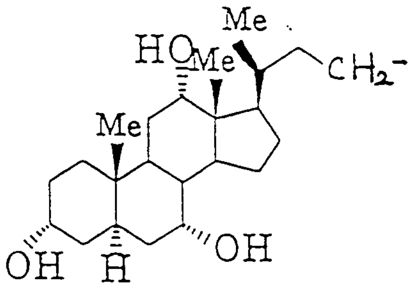

(B)

or -OH, $R_2$ is a group represented by the formula (A), -OH or -H and each of $R_5$ and $R_6$ is -H, $-CH_2OH$, $-CH_3$ or $-CH_2OSO_3Na$, in which $R_2$ and $R_5$ may combine with each other to be $-OCH_2-$, $R_2$ and $R_6$ may combine with each other to be $-OCH_2-$ and $R_5$ and $R_6$ may combine with each other to be $-CH_2OCH_2-$, provided that $R_5$ and $R_6$ are not -H at the same time.

**12.** An anti-thrombotic agent which comprises a bile alcohol as the active ingredient represented by the following general formula (II):

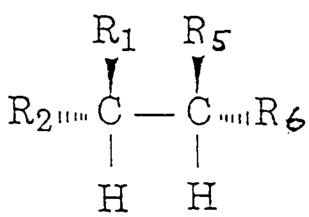

(II)

**13.** An anti-hypertensive agent which comprises a bile alcohol as the active ingredient represented by the following general formula (II):

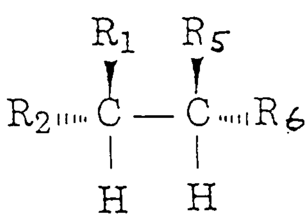

(II)

**14.** A cerebral function protector which comprises a bile alcohol as the active ingredient represented by the following general formula (III):

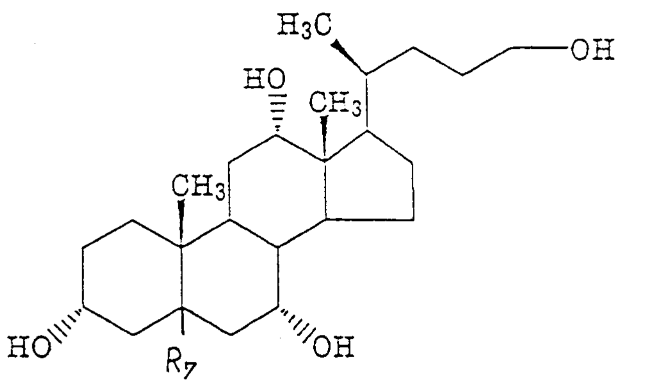

(III)

wherein $R_7$ is α-H or β-H.

**15.** A cholagogue which comprises a bile alcohol as the active ingredient represented by the following general formula (III):

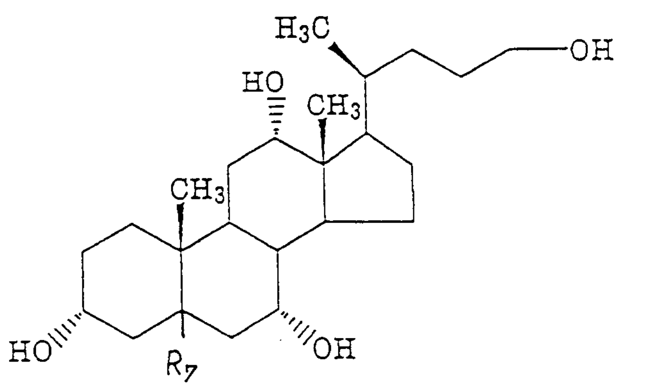

(III)

wherein $R_7$ is α-H or β-H.

**16.** A cell activator which comprises a bile alcohol as the active ingredient represented by the following general formula (III):

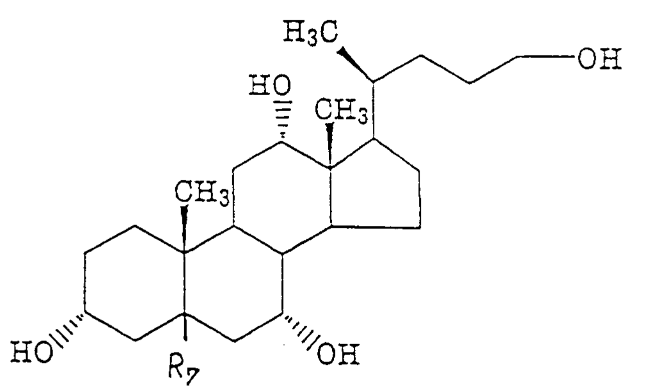

(III)

wherein $R_7$ is α-H or β-H.

**17.** A microcirculation improver which comprises a bile alcohol as the active ingredient represented by the following general formula (III):

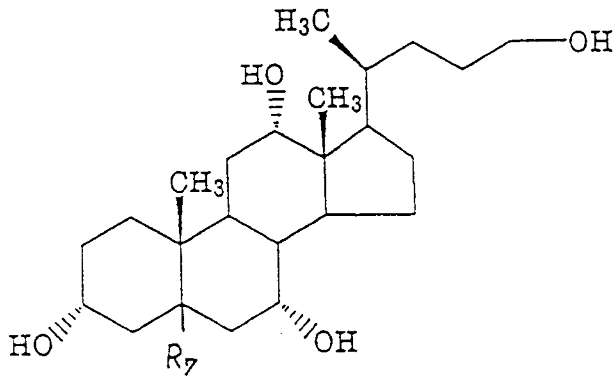

(III)

wherein $R_7$ is $\alpha$-H or $\beta$-H.

**18.** An anti-arteriosclerotic agent which comprises a bile alcohol as the active ingredient represented by the following general formula (III):

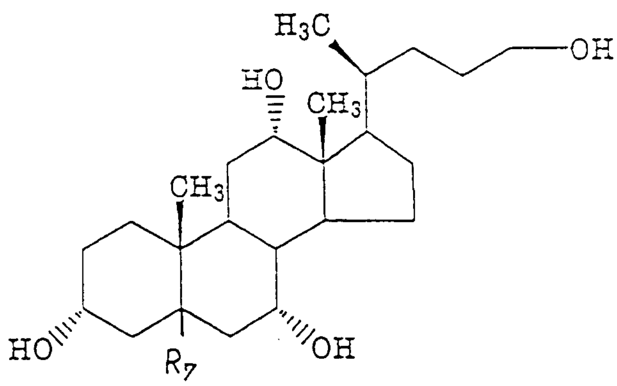

(III)

wherein $R_7$ is $\alpha$-H or $\beta$-H.

**19.** An anti-hyperlipemic agent which comprises a bile alcohol as the active ingredient represented by the following general formula (III):

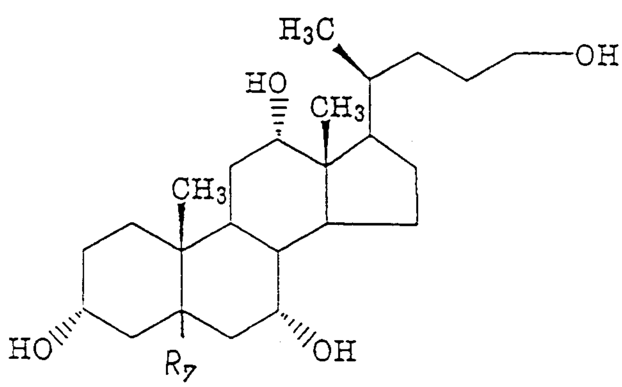

(III)

wherein $R_7$ is $\alpha$-H or $\beta$-H.

**20.** A liver function improver which comprises a bile alcohol as the active ingredient represented by the following general formula (III):

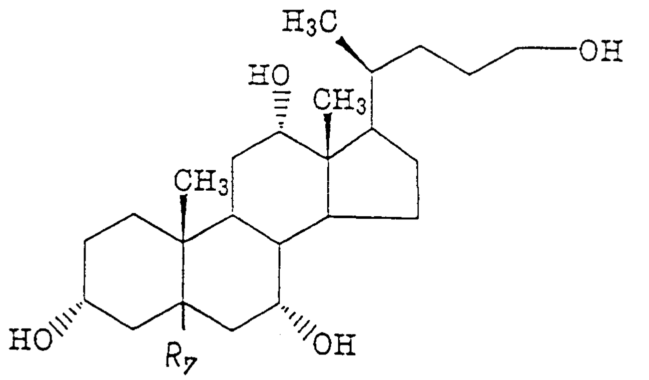

(III)

wherein $R_7$ is α-H or β-H.

**21.** An anti-inflammatory agent which comprises a bile alcohol as the active ingredient represented by the following general formula (III):

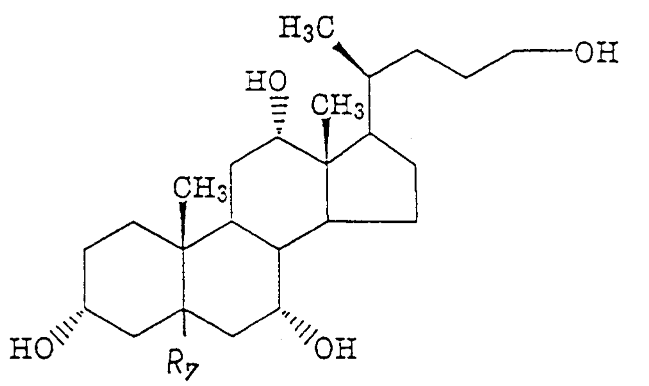

(III)

wherein $R_7$ is α-H or β-H.

**22.** An anti-thrombotic agent which comprises a bile alcohol as the active ingredient represented by the following general formula (III):

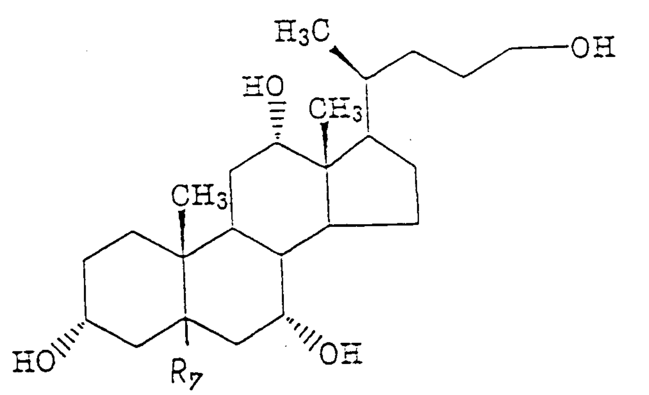

(III)

wherein $R_7$ is α-H or β-H.

**23.** An anti-hypertensive agent which comprises a bile alcohol as the active ingredient represented by the following general formula (III):

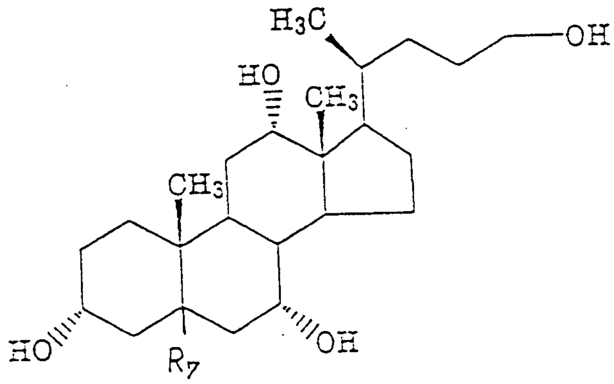

(III)

wherein $R_7$ is $\alpha$-H or $\beta$-H.

# INTERNATIONAL SEARCH REPORT

International Application No PCT/JP91/00888

**I. CLASSIFICATION OF SUBJECT MATTER** (if several classification symbols apply, indicate all) [6]

According to International Patent Classification (IPC) or to both National Classification and IPC

Int. $Cl^5$ C07J9/00, A61K31/575

**II. FIELDS SEARCHED**

Minimum Documentation Searched [7]

| Classification System | Classification Symbols |
|---|---|
| IPC | C07J9/00, A61K31/575 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | WO, A1, 8,907,607 (Broadbent, James Meredyth), August 24, 1989 (24. 08. 89) | 1-3, 5, 10, 15, 20 |
| A | US, A, 3,931,403 (Intellectual Proporty Development Corporation), January 6, 1976 (06. 01. 76), (Family: none) | 1-3 |
| A | US, A, 4,029,775 (Intellectual property Development Corporation), June 14, 1977 (14. 06. 77), (Family: none) | 1-3 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| August 30, 1991 (30. 08. 91) | September 17, 1991 (17. 09. 91) |
| **International Searching Authority** | **Signature of Authorized Officer** |
| Japanese Patent Office | |